# EUROPEAN PATENT SPECIFICATION

(11) **EP 3 471 697 B1**
(45) Date of publication and mention of the grant of the patent: **04.09.2024**
(21) Application number: 17736703.4
(22) Date of filing: 15.06.2017
(51) Int. Cl.: A61K 9/00, A61K 9/14, A61K 9/19, A61K 31/122, A61P 11/00, A61P 33/00, A61P 31/10, A61P 33/02, A61P 33/06, A61P 33/08

(54) **CHEMICAL COMPOSITION**
CHEMISCHE ZUSAMMENSETZUNG
COMPOSITION CHIMIQUE

(30) Priority: 16.06.2016 US 201662351048 P
(43) Date of publication of application: 24.04.2019
(73) Proprietor: The University of Liverpool, Liverpool, Merseyside L69 7ZX (GB); The Johns Hopkins University, Baltimore, Maryland 21218 (US)
(72) Inventor: RANNARD, Steven Paul, Liverpool Merseyside L69 3GL (GB); OWEN, Andrew, Liverpool Merseyside L69 3GL (GB); SAVAGE, Alison, Liverpool Merseyside L69 3GL (GB); TATHAM, Lee, Liverpool Merseyside L69 3GL (GB); SHAPIRO, Theresa, Baltimore, Maryland 21218 (US); BAKSHI, Rahul P., Baltimore, Maryland 21218 (US); MLAMBO, Godfree, Baltimore, Maryland 21218 (US); TRIPATHI, Abhai, Baltimore, Maryland 21218 (US)
(74) Representative: Marks & Clerk LLP
(86) International application number: PCT/GB2017/051746
(87) International publication number: WO 2017/216564

(56) References cited:
- WO-A1-94/14426
- US-A1- 2010 028 425
- N. SCHOLER ET AL: "Atovaquone Nanosuspensions Show Excellent Therapeutic Effect in a New Murine Model of Reactivated Toxoplasmosis", ANTIMICROBIAL AGENTS AND CHEMOTHERAPY, vol. 45, no. 6, 1 June 2001 (2001-06-01), pages 1771 - 1779, XP055400820, ISSN: 0066-4804, DOI: 10.1128/AAC.45.6.1771-1779.2001
- DALENCON F ET AL: "Atovaquone and rifabutine-loaded nanocapsules: formulation studies", INTERNATIONAL JOURNAL OF PHARMACEU, ELSEVIER, AMSTERDAM, NL, vol. 153, no. 1, 1 January 1997 (1997-01-01), pages 127 - 130, XP009103321, ISSN: 0378-5173, DOI: 10.1016/S0378-5173(97)00076-8

## Description

The present invention relates to a chemical composition. The invention relates, more particularly, but not exclusively, to a chemical composition for the treatment and prophylaxis of systemic infections, and has particular (but not exclusive) application in the treatment and prophylaxis of systemic parasitic and fungal infections, such as malaria, toxoplasmosis, and *Pneumocystis* pneumonia.

Taking malaria as an example of a parasitic infection for which both an improved treatment and an improved prophylactic offering would be desirable, the reader will no doubt be aware that malaria is a vector-borne infectious disease that is widespread in tropical and subtropical regions. Over 200 million people are infected with malaria and there are almost 500,000 deaths annually, mostly among young children in sub-Saharan Africa. Furthermore, literature indicates that tens of millions of travellers per year required malaria prophylaxis (Leder K. et al., Clin. Infect Dis., 2004, 39, 1104-1112). Malaria is caused by protozoan of the genus *Plasmodium.* The most serious form of the disease is caused by *P. falciparum* but other related species *P. vivax, P. ovale, P. malariae* and *P. knowlesi* can infect humans. This group of human-pathogenic *Plasmodium* species is usually referred to as the malaria parasites.

Although currently there is no effective vaccine against malaria, effective oral preparations for the treatment and prophylaxis of malaria have been known for many years. For example, a combination preparation of atovaquone with proguanil hydrochloride is available under the trade name Malarone^{™}, with a standard (adult) tablet containing 250 mg of atovaquone and 100 mg of proguanil hydrochloride. To prevent malaria in an adult (weighing over 40 kg), one standard tablet should be taken once a day 24 to 48 hours before entering a malarial area, continuing with one tablet once a day for the duration of stay in the malarial area, and further continuing for 7 days after leaving the malarial area, .i.e. 2.25 to 2.5 g of atovaquone for just a one-day stay in a malarial area. To treat malaria in an adult, four standard tablets, should be taken every day for three days, i.e. 3 g of atovaquone in total.

Atovaquone is a hydroxy-1,4-naphthoquinone and is an antiprotozoal agent. It exists as a yellow crystalline solid that is practically insoluble in water, but is highly lipophilic. The structure of atovaquone is shown below.

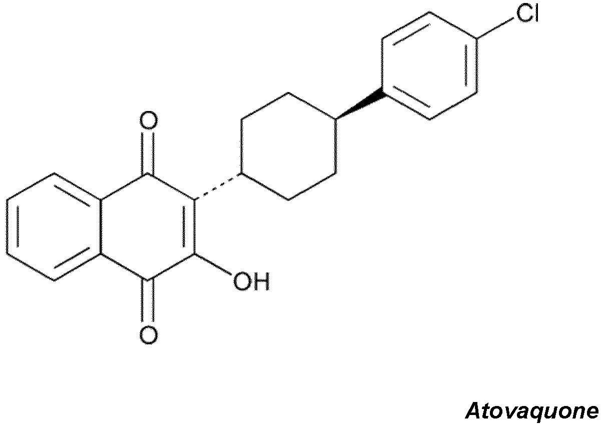

For the treatment of severe malaria, parenteral (or rectal) preparations of artesunate (a derivative of artemisinin) is recommended (as of 2011) by the World Health Organisation, until oral medication can be tolerated by the patient. Artemisinin is a naturally occurring sesquiterpene lactone, the structure of which is shown below. Artemisinin-derived preparations are among the substances which are currently believed to act most rapidly against malaria parasites, and thus are preferred for the treatment of severe malaria.

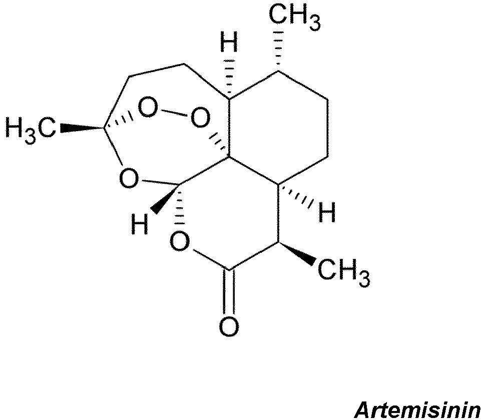

However, in countries such as the USA where, e.g. intravenous artesunate, is not approved for use, intravenous quinidine may be used. The structure of quinidine is shown below.

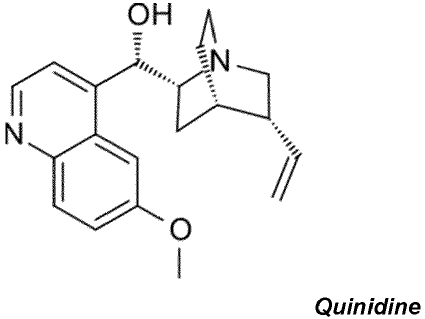

Other currently available preparations for (a) the propylaxis and (b) the treatment of malaria include: (a) doxycycline, mefloquine, chloroquine and primaquine, and (b) coartem (artemether plus lumefantrine), quinine, and chloroquine, respectively.

The effective prophylaxis, control and treatment of malaria presents enormous challenges, especially in sub-Saharan Africa where access to medicines and health care is limited. Poor adherence to malaria prophylaxis regimes and antimalarial treatment, from both health workers and consumers, has been associated with prophylaxis/treatment failure, severe malaria and death. Poor adherence leads to sub-therapeutic drug concentrations in the body, which will not eradicate all malaria parasites and may allow growth of resistant parasites. Drug concentrations in the body can become sub-therapeutic either by missed doses and/or by doses being taken but not in a timely manner. Thus, high levels of patient adherence to malaria prophylactic regimes and antimalarial treatment are important in ensuring effectiveness of the prescribed preparations.

US 2010/028425 A1 describes immediate release pharmaceutical compositions for oral administration comprising micronized atovaquone particles and at least one drug, wherein about 90% of the atovaquone particles have a volume diameter between about 4 µm to about 8 µm; and having a uniform release profile after a storage for at least three months at 40° C. and 75% relative humidity.

WO 94/14426 A1 describes microfluidised particles of atovaquone and a method of preparing them, as well as a pharmaceutical composition containing microfluidised particles of atovaquone which has improved bioavailability and its use in therapy.

N. Scholer et al ("Atovaquone Nanosuspensions Show Excellent Therapeutic Effect In A New Murine Model Of Reactivated Toxoplasmosis", Antimicrobial Agents and Chemotherapy, vol.45, no. 6, 1 June 2001, pages 1771-1779) describe atovaquone nanosuspensions (ANSs) for intravenous (i.v.) administration. Development of toxoplasma encephalitis and mortality in mice treated with 1.0- or 0.1-mg/kg i.v. doses of ANS did not differ from that in mice treated orally with 100 mg of atovaquone/kg.

F. Dalencon et al ("Atovaquone and rifabutine-loaded nanocapsules: formulation studies", International Journal of Pharmaceutics, vol. 153, no.1, 1 January 1997, pages 127-130) describe the formulation of atovaquone as a colloidal suspension of poly (D,L) lactic acid nanocapsules. A better survival rate was observed in mice treated with the nanocapsules than in mice treated with a suspension of the drug.

An improved manner of treating and/or preventing malaria would therefore be desirable, from any one or more of the points of view of reduction in dosing, ease of administration, increased patient adherence/compliance and simplification of follow-up care.

An improved manner of treating and/or preventing any of the following other parasitic and fungal diseases, such as toxoplasmosis (caused by the parasite *Toxoplasma gondii*)*,* babesiosis (caused by the parasite *Babesia microti*) and *Pneumocystis* pneumonia (caused by the fungus *Pneumocystis jirovecii*)*,* as briefly mentioned above, would also be desirable for many of the same reasons as provided for the desire to improve the treatment and/or prophylaxis of malaria.

It is therefore an object of the present invention to obviate and/or mitigate at least one of the disadvantages associated with existing antiparasitic and antifungal prophylactic and/or treatment offerings, whether identified herein or otherwise.

It is furthermore an object of the present invention to provide an improved chemical composition for prophylaxis and/or treatment of parasitic and fungal infections, especially malaria, toxoplasmosis, babesiosis and *Pneumocystis* pneumonia.

According to a first aspect of the present invention there is provided a solid composition comprising nanoparticles of atovaquone dispersed within one or more carrier materials, wherein the atovaquone is present in an amount of at least 10 wt%, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate; and
- polyvinyl alcohol AND polyethylene glycol (15)-hydroxystearate.

For the avoidance of any doubt, the solid composition according to the first aspect of the invention comprises solid nanoparticles of atovaquone dispersed within one or more solid carrier materials. More than one solid carrier material is present, said two or more solid carrier materials are present in a mixture, in which the solid nanoparticles of atovaquone are dispersed. The one or more solid carrier materials effectively form a matrix in which the solid nanoparticles of atovaquone are dispersed. The chemical composition of the invention is therefore a mixture of substances, and all references to "chemical composition" or "solid composition" in this specification should be construed accordingly.

According to a second aspect of the present invention there is provided an aqueous dispersion, comprising a plurality of nanoparticles of atovaquone dispersed in an aqueous medium, each nanoparticle of atovaquone being a core around at least some of which an outer layer composed of one or more carrier materials may be provided, wherein the atovaquone is present in a concentration of at least 10 mg/mL, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

Throughout this specification, by "may be provided" in relation to the outer layer, it is meant that some of the nanoparticles of atovaquone will be provided with said outer layer, but not all of the nanoparticles of atovaquone need be provided with said outer layer, although all could be.

For the avoidance of any doubt, the aqueous dispersion according to the second aspect of the invention comprises a plurality of solid nanoparticles of atovaquone dispersed in an aqueous medium, each solid nanoparticle of atovaquone being a core around which an outer layer (which need not be a uniform layer in terms of its thickness, composition and/or any other properties) composed of one or more carrier materials may be provided.

According to a third aspect of the present invention there is provided an oily dispersion, comprising a plurality of nanoparticles of atovaquone and one or more carrier materials dispersed in an oily medium, wherein the atovaquone is present in a concentration of at least 10 mg/mL, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

Said dispersion may range in physical composition from (a) the one or more carrier materials thereof being completely dissolved or dispersed in the oily medium (suitable non-aqueous diluent) and the solid nanoparticles of atovaquone being dispersed in the oily medium, to (b) smaller particles of a matrix of the one or more carrier materials containing some nanoparticles of atovaquone being dispersed in the oily medium such that the nanoparticles of atovaquone remain trapped in the matrix, i.e. the matrix per se does not dissolve.

Dependent on the ratio of solid materials, i.e. atovaquone and the one or more carrier materials, to oily medium - which can be varied accordingly - the oily dispersion may be in the form of a paste having a high ratio of solid materials to oily medium.

Throughout this specification, unless stated otherwise, use of the term "dispersion(s)" as a noun and like terms means both the aqueous dispersion and the oily dispersion of the invention.

According to a fourth aspect of the present invention there is provided an intramuscularly-injectable formulation of nanoparticles of atovaquone, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

According to a fifth aspect of the present invention there is provided a subcutaneously-injectable formulation of nanoparticles of atovaquone, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate..

In other words, the invention also provides both a stable, directly intramuscularly-injectable formulation of atovaquone and a stable, directly subcutaneously-injectable formulation of atovaquone, each of which may be in solid form (or substantially solid form, e.g. a paste) or liquid form, in which the atovaquone is present in the form of nanoparticles.

Throughout this specification, unless stated otherwise, use of the term "injectable formulation(s)" and like terms means both the intramuscularly-injectable formulation and the subcutaneously-injectable formulation.

Each of the solid composition, the dispersions and the injectable formulations of the present invention comprise the atovaquone in solid nanoparticulate form. The nanoparticles of the present invention provide improved dosage forms of atovaquone. Each of the solid composition, the dispersions and the injectable formulations of the present invention also provide a means of achieving systemic distribution of atovaquone in the body and provide higher drug levels in the circulating bloodstream. These advantages provide the opportunity for a more effective treatment and/or prophylaxis of systemic infection, especially systemic parasitic and fungal infections. Alternatively or additionally, these advantages may also enable the required dosage of atovaquone to be reduced.

The provision of nanoparticles in a solid composition can also be advantageous because they can be dispersed in a suitable aqueous or oily diluent (e.g. a pharmaceutically- or veterinary-acceptable injectable oil) when required to form an aqueous dispersion or an oily dispersion respectively of the nanoparticles for administration, e.g. parenterally. Such dispersion of a solid composition may form the injectable formulations of the invention.

When the oily dispersion is formed by dispersion of a solid composition in a suitable oily diluent, said dispersion may range in physical composition from (a) the one or more carrier materials thereof completely dissolving or dispersing in the oily diluent such that the nanoparticles of atovaquone are released and dispersed in the oily diluent, to (b) smaller particles of the composition, e.g. formed by milling or grinding or other suitable comminution techniques applied to the bulk solid composition, being dispersed in the oily diluent. In the latter case, the smaller particles of the composition that are dispersed in the oily diluent are effectively discrete portions of matrix of one or more carrier materials containing some nanoparticles of atovaquone; the matrix per se does not dissolve and the nanoparticles of atovaquone remain trapped in the matrix.

Equally, the provision of a ready-to-use injectable formulation - whether in solid form or liquid form - provides a simple and convenient form of medication that is easily administered.

Both the solid compositions and dispersions of the present invention are ideally suited to personalised medicine regimes, because both are (or can be made in the case of a dispersion) substantially homogeneous, meaning that partial doses of atovaquone may be accurately measured, as needed. Furthermore, the solid compositions of the present invention are readily dispersible within an aqueous medium or an oily medium to provide stable aqueous dispersions or oily dispersions respectively. Such stable dispersions can themselves be partitioned in a pre-determined manner to provide an accurate liquid dose of atovaquone. Accurate dosing of atovaquone is also achievable with the injectable formulations of the present invention, whether in solid or liquid form.

Such methods of providing personalised doses are particularly applicable to paediatric administration, since children require lower doses. Moreover, atovaquone doses can be accordingly adapted to suit a patient's weight, age, and other circumstances (such as the stage or severity of the parasitic or fungal infection).

According to a sixth aspect of the present invention there are provided processes for the preparation of a solid composition as defined herein.

According to a seventh aspect of the present invention, there is provided a pharmaceutical composition comprising a solid composition of the first aspect of the invention, or an aqueous dispersion of the second aspect of the invention, or an oily dispersion of the third aspect of the invention, and optionally a further pharmaceutically acceptable diluent, carrier, or excipient. Said composition may also be a veterinary composition.

According to an eighth aspect of the present invention, there is provided a solid composition, a dispersion, or an injectable formulation as defined herein for use as a medicament.

According to a ninth aspect of the present invention, there is provided a solid composition, a dispersion, or an injectable formulation as defined herein for use in the treatment and/or prevention of parasitic and fungal infections (e.g. malaria, toxoplasmosis, babesiosis, *Pneumocystis* pneumonia).

According to a tenth aspect of the present invention, there is provided a kit for the preparation of a sterile liquid formulation of atovaquone for injection, the kit comprising: a first container comprising a solid composition, a pharmaceutical or veterinary injectable composition, or an injectable formulation as defined herein; and a second container comprising a sterile aqueous or oily (non-aqueous) diluent in an amount sufficient to dilute the atovaquone to a concentration of at least 10 mg/mL.

For the avoidance of any doubt, features (including optional, suitable and preferred features) of any aspect of the invention are to be considered, unless otherwise stated, as also being features (including optional, suitable and preferred features) of any and all other aspect(s) of the invention.

### DEFINITIONS

The term "nanoparticle" or "nanoparticulate" is used herein to mean a particle having a size of less than or equal to 1 micron (µm), but greater than or equal to 1 nanometre (nm), i.e. in the range 1-1000 nm. These terms are clear and well understood by a person skilled in the art, without any confusion, not least as evidenced by Petros and DeSimone, Nature Reviews Drug Discovery, 2010, 9, 615-627.

Unless otherwise stated, the term "particle size" is used herein to refer to the number average particle diameter.

Unless otherwise stated, the term "patient" includes both human patients and animal patients.

The term "atovaquone" is used herein to refer to the molecule illustrated in the background section as being atovaquone, and also includes pharmaceutically acceptable salts, solvates and derivatives thereof, prodrugs thereof, as well as any polymorphic or amorphous forms thereof.

The term "other drugs" is used herein to refer to the following (non-exhaustive) list of other drugs that may be used in combination with atovaquone formulated in accordance with the invention in a combination prophylactic and/or treatment therapy: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine, as well as pharmaceutically acceptable salts, solvates and derivatives thereof, prodrugs thereof, and any polymorphic or amorphous forms thereof.

It is to be appreciated that references to "preventing" or "prevention" relate to prophylactic treatment and includes preventing or delaying the appearance of clinical symptoms of the state, disorder or condition developing in a patient that may be afflicted with or predisposed to the state, disorder or condition but does not yet experience or display clinical or subclinical symptoms of the state, disorder or condition. With specific reference to malaria, because malaria parasites are confined to the circulating bloodstream (in red blood cells), or for the first few days of infection, to the liver, the prophylactic effect of atovaquone extends to both the initial liver stage (causal prophylaxis) and the red blood cell stage (suppressive prophylaxis).

It will be further appreciated that references to "treatment" or "treating" of a state, disorder or condition includes: (1) inhibiting the state, disorder or condition, *i.e.,* arresting, reducing or delaying the development of the disease or a relapse thereof (in case of maintenance treatment) or at least one clinical or subclinical symptom thereof; or (2) relieving or attenuating the disease, *i.e.* causing regression of the state, disorder or condition or at least one of its clinical or subclinical symptoms.

In the context of the invention, the terms "preventing" or "prevention" should not be considered to refer only to medicaments which are completely effective in treating a specific state, disorder or condition, but also to cover medicaments which are partially effective as well.

Moreover, when considered from the perspective of a population of patients for treatment, the terms "preventing" and "prevention" should be considered to cover medicaments which are useful at reducing the rate of incidence of a target disorder or condition (e.g. malaria, toxoplasmosis, babesiosis, *Pneumocystis* pneumonia) in that target population, as well as medicaments which are useful at completely eradicating a target state, disorder or condition from that target population.

A "therapeutically effective amount" means the amount of a compound that, when administered to a patient for treating and/or preventing a disease, is sufficient to effect such treatment/prevention for the disease. The "therapeutically effective amount" will vary depending on the compound, the disease and its severity and the age, weight, etc., of the patient to be treated.

The term "consisting essentially of" is used herein to denote that a given product or method consists of only designated materials or steps and optionally other materials or steps that do not materially affect the characteristic(s) of the claimed invention. Suitably, a product which consists essentially of a designated material (or materials) comprises greater than or equal to 85% of the designated material, more suitably greater than or equal to 90%, more suitably greater than or equal to 95%, most suitably greater than or equal to 98% of the designated material(s).

Unless otherwise stated, the weight percentages ("wt%") discussed herein relate to the % by weight of a particular constituent as a proportion of the total weight of the composition.

References herein to a carrier material being "(substantially) immiscible" with another carrier material means that a mixture comprising the two carrier materials is unable to form a single phase.

### SOLID COMPOSITION

The present invention provides a solid composition comprising nanoparticles of atovaquone dispersed within one or more carrier materials, wherein the atovaquone is present in an amount of at least 10 wt%.

Preferably, the atovaquone is present in an amount of at least 15 wt%, further preferably at least 20 wt%, yet further preferably at least 25 wt%, yet further preferably at least 30 wt%, yet further preferably at least 40 wt%, yet further preferably at least 50 wt%, yet further preferably at least 60 wt%, yet further preferably 70 wt%, and most preferably at least 80 wt%.

Accordingly, the one or more solid carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

Accordingly, the one or more solid carrier material may be provided in any one or more of the following combinations:
- polyvinyl alcohol-polyethylene glycol graft copolymer AND polyoxyethylene (20) sorbitan monooleate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinyl alcohol AND sodium deoxycholate.

The one or more solid carrier materials are preferably water-soluble. In the context of the present invention, "water-soluble" as applied to the carrier material(s) means that its solubility in water at ambient temperature and pressure is at least 10g/L.

Preferably, individual solid nanoparticles of atovaquone consist essentially of atovaquone.

The solid composition of the present invention may be administered as it is to a patient, or further formulated to provide a pharmaceutical composition in the form of, for example, an injectable formulation, such as an intramuscular depot injection or a subcutaneous depot injection.

The nanoparticles of atovaquone have an average particle size of less than or equal to 1 micron (µm). Preferably, the nanoparticles of atovaquone have an average particle size of between 100 and 1000 nm. Further preferably, the nanoparticles of atovaquone have an average particle size between 200 and 850 nm.

Particle sizes may be assessed by any suitable technique known in the art (e.g. laser diffraction, laser scattering, electron microscopy). Particle sizes herein have been assessed by dispersing the solid composition in an aqueous medium and determining the particle size by dynamic light scattering with a Zetasizer (Malvern Instruments Ltd).

The polydispersity of the nanoparticles of atovaquone may be less than or equal to 0.8, preferably less than or equal to 0.6, and more preferably less than or equal to 0.4. The polydispersity relates to the size of the atovaquone nanoparticles and may be determined by suitable techniques known in the art (e.g. laser diffraction, laser scattering, electron microscopy). Polydispersity of particle sizes of the nanoparticles of atovaquone herein have been assessed with a Malvern Zetasizer (Malvern Instruments Ltd).

The average zeta potential of the nanoparticles of atovaquone when dispersed in an aqueous medium may be between -100 and +100 mV. In one embodiment, the zeta potential of the nanoparticles of atovaquone may be between -30 and +30 mV. In another embodiment, the zeta potential of the nanoparticles of atovaquone may be between -25 and +25 mV. In yet another embodiment, the zeta potential of the nanoparticles of atovaquone may be between -25 and +10 mV. In general it has been found that zeta potentials of a relatively small magnitude (either positive or negative) allow the nanoparticles to better penetrate into and accumulate within cells. In accordance with the present invention, average zeta potentials can be measured by techniques known in the art, such as using a Zetasizer (Malvern Instruments Ltd).

The solid composition may comprise solid particles or granules of larger size, for example, 5 to 30 microns (µm) in size, wherein each particle or granule contains a plurality of solid nanoparticles of atovaquone dispersed within the one or more solid carrier materials. These larger particles or granules disperse when the solid composition is mixed with an aqueous medium to form discrete solid nanoparticles of atovaquone.

### Hydrophilic Polymer

The following hydrophilic polymers are suitable for use in the present invention:
- polyvinyl alcohol-polyethylene glycol graft copolymer (available under the trade name Kollicoat^{™});
- polyvinyl alcohol ('PVA');
- polyvinylpyrrolidone k30, having average M_{w} of 30,000 (`PVP k30').

### Surfactant

The following surfactants are suitable for use in the present invention:
- polyoxyethylene (20) sorbitan monolaurate, also known as polysorbate 20 (available under the trade name Tween^{™} 20);
- polyoxyethylene (20) sorbitan monooleate, also known as polysorbate 80 (available under the trade name Tween^{™} 80);
- sodium deoxycholate;
- D-α-tocopherol polyethylene glycol 1000 succinate;
- polyethylene glycol (15)-hydroxystearate (available under the trade name Solutol^{™} HS).

In particular, the following surfactants are preferred for use in the present invention:
- polyoxyethylene (20) sorbitan monolaurate, also known as polysorbate 20 (available under the trade name Tween^{™} 20);
- polyoxyethylene (20) sorbitan monooleate, also known as polysorbate 80 (available under the trade name Tween^{™} 80);
- sodium deoxycholate;
- D-α-tocopherol polyethylene glycol 1000 succinate.

In the present invention, the solid carrier material having surfactant activity is suitably selected from those surfactants that are capable of stabilising nanoparticles of atovaquone together with the carrier material having hydrophilic polymeric activity as defined herein, and which are also approved for pharmaceutical use (e.g. they are approved for use by the US Food and Drug Administration).

### Particular Combinations of Hydrophilic Polymer and Surfactant

The following combinations of hydrophilic polymer(s) and surfactant(s) are used in the present invention:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

### Formulation of the Solid Composition

In a particular embodiment, the solid composition as defined herein may comprise 10 to 99 wt% of atovaquone. Preferably, or in another embodiment, the solid composition may comprise 15 to 95 wt% of atovaquone. Further preferably, or in another embodiment, the solid composition may comprise at least 20 wt%, more preferably or alternatively at least 40 wt%, yet further preferably or alternatively at least 60 wt%, and most preferably or alternatively at least 80 wt% of atovaquone.

The atovaquone is present in any amount of at least 10 wt%, such as in the range of from 10-99 wt%, or from 10-95 wt%, or from 10-90 wt%.

Alternatively, the atovaquone may be present in an amount of at least 15 wt%, such as in the range of from 15-99 wt%, or from 15-95 wt%, or from 15-90 wt%.

Further alternatively, the atovaquone may be present in an amount of at least 20 wt%, such as in the range of from 20-99 wt%, or from 20-95 wt%, or from 20-90 wt%.

Yet further alternatively, the atovaquone may be present in an amount of at least 25 wt%, such as in the range of from 25-99 wt%, or from 25-95 wt%, or from 25-90 wt%.

Still further alternatively, the atovaquone may be present in an amount of at least 30 wt%, such as in the range of from 30-99 wt%, or from 30-95 wt%, or from 30-90 wt%.

Still further alternatively, the atovaquone may be present in an amount of at least 40 wt%, such as in the range of from 40-99 wt%, or from 40-95 wt%, or from 40-90 wt%.

Still further alternatively, the atovaquone may be present in an amount of at least 50 wt%, such as in the range of from 50-99 wt%, or from 50-95 wt%, or from 50-90 wt%.

Still further alternatively, the atovaquone may be present in an amount of at least 60 wt%, such as in the range of from 60-99 wt%, or from 60-95 wt%, or from 60-90 wt%.

Still further alternatively, the atovaquone may be present in an amount of at least 70 wt%, such as in the range of from 70-99 wt%, or from 70-95 wt%, or from 70-90 wt%.

Still further alternatively, the atovaquone may be present in an amount of at least 80 wt%, such as in the range of from 80-99 wt%, or from 80-95 wt%, or from 80-90 wt%.

The solid compositions of the present invention therefore permit high drug loadings, which keeps the potentially toxic excipients (e.g. surfactants) to a minimum.

The solid composition may comprise 1 to 90 wt% of the one or more selected carrier materials. Preferably, or in one embodiment, the solid composition may comprise 5 to 85 wt% of the one or more carrier materials. Further preferably, or in another embodiment, the solid composition may comprise 10 to 80 wt% of the one or more carrier materials. In a particular embodiment, the solid composition may comprise 20 to 60 wt% of the one or more selected carrier materials. For the avoidance of doubt, the percentage amount of the selected carrier materials refers to the total weight amount of all said selected carrier materials in that composition.

In a particular embodiment, the solid composition may comprise 1 to 90 wt% of hydrophilic polymer. Preferably, or in another embodiment, the solid composition may comprise 8 to 70 wt% of hydrophilic polymer. Further preferably, or in another embodiment, the solid composition may comprise 10 to 60 wt% of hydrophilic polymer. Yet further preferably, or in a particular embodiment, the solid composition may comprise 10 to 50 wt% of hydrophilic polymer.

In a particular embodiment, the solid composition may comprise 1 to 70 wt% of surfactant. Preferably, or in another embodiment, the solid composition may comprise 2 to 50 wt% of surfactant. Further preferably, or in another embodiment, the solid composition may comprise 3 to 30 wt% of surfactant.

In an embodiment, the solid composition may comprise the carrier material providing hydrophilic polymeric activity and the carrier material providing surfactant activity in a respective ratio of between 30:1 and 1:10. Preferably, or in a particular embodiment, the solid composition may comprise the carrier material providing hydrophilic polymeric activity and the carrier material providing surfactant activity in a respective ratio of between 15:1 and 1:2. Further preferably, or in another embodiment, the solid composition may comprise the carrier material providing hydrophilic polymeric activity and the carrier material providing surfactant activity in a respective ratio of between 10:1 and 2:1. Yet further preferably, or in a particular embodiment, the solid composition may comprise the carrier material providing hydrophilic polymeric activity and the carrier material providing surfactant activity in a respective ratio of between 6:1 and 3:1.

In a particularly preferred embodiment, the solid composition comprises:
- 40 to 90 wt% atovaquone;
- 8 to 40 wt% of a carrier material providing hydrophilic polymeric activity; and
- 2 to 20 wt% of a carrier material providing surfactant activity.

The solid composition may comprise one or more additional excipients, for instance, to further facilitate dispersion or stabilisation of dispersions of the nanoparticles either in a pharmaceutically acceptable diluent or *in vivo.*

### Processes for Preparing the Solid Composition

Solid compositions of the present invention may be prepared by a number of methods well known in the art. Suitable techniques for forming such compositions are described in general terms in Horn and Rieger, Angew. Chem. Int. Ed., 2001, 40, 4330-4361.

For example, the solid composition may be prepared by milling a solid form of atovaquone. The milling may occur in the presence of the one or more carrier materials, e.g. the hydrophilic polymer and surfactant, or, alternatively, they may be mixed with the milled drugs after the milling step.

However, it is generally preferred that the solid atovaquone compositions of the present invention are prepared by an oil-in-water (o/w) emulsion technique whereby the atovaquone is dissolved in the oil phase and the carrier material(s), e.g. providing hydrophilic polymeric and surfactant activity, are present in the water phase. The oil and water solvents are then removed by freeze-drying, spray-drying or spray-granulation to provide a solid composition according to the invention.

Thus, in accordance with one aspect of the present invention, there is provided a process for preparing a solid composition as defined herein, the process comprising:
(a) preparing an oil-in-water emulsion comprising:
   - an oil phase comprising atovaquone; and
   - an aqueous phase comprising one or more selected carrier materials as defined herein; and
(b) removing the oil and water from the oil-in-water emulsion to form the solid composition.

An advantage of the processes of the present invention is that the emulsions formed in the initial steps are sufficiently homogenous and stable to allow for effective and uniform drying upon removal of the oil and water. Furthermore, the nanoparticles formed are substantially uniform in their physical form (size, shape etc.).

The oil-in-water emulsion formation steps may be performed by methods well-known in the art. Any suitable method for forming the oil-in-water emulsions may therefore be used. In particular, mixing of the oil and water phases to form the oil-in-water emulsion may be performed by methods well known in the art. For example, the mixing may involve stirring, sonication, homogenisation, or a combination thereof. In a particular embodiment, the mixing is facilitated by sonication and/or homogenisation.

The oil-in-water emulsion formation steps may be performed, for example, by using the methods described in WO 2004/011537 A1 (COOPER et al)*.*

In a particular embodiment, oil-in-water emulsion formation comprises:
(i) providing an oil phase comprising atovaquone;
(ii) providing an aqueous phase comprising the one or more selected carrier materials; and
(iii) mixing the oil phase and aqueous phase to produce the oil-in-water emulsion.

Suitably, the oil phase is provided by dissolving atovaquone in a suitable organic solvent. Suitably, the aqueous phase is provided by dissolving the one or more selected carrier materials in an aqueous medium, preferably in water. In embodiments where more than one said selected carrier material is used, the aqueous phase may be provided by mixing a corresponding number of separately prepared aqueous solutions of each selected carrier material.

In a particular embodiment, further aqueous medium (e.g. water) or organic solvent is added before or during mixing step (iii).

The concentration of atovaquone in the oil-in-water emulsion is suitably as concentrated as possible to facilitate effective scale-up of the process. For example, the concentration of drug(s) in the oil phase is suitably 10 mg/mL or higher, more suitably 15 mg/mL or higher, even more suitably greater than 20 mg/mL or higher.

The concentration of the carrier material providing hydrophilic polymeric activity in the aqueous/water phase is suitably 0.5 to 50 mg/mL.

The concentration of the carrier material providing surfactant activity in the aqueous/water phase emulsion is suitably 0.5 to 50 mg/mL.

The organic solvent forming the oil phase is (substantially) immiscible with water. Suitably the organic solvent is aprotic. Suitably the organic solvent has a boiling point less than 120°C, suitably less than 100°C, suitably less than 90°C.

In a particular embodiment, the organic solvent is a selected from the Class 2 or 3 solvents listed in the International Conference on Harmonization (ICH) guidelines relating to residual solvents.

In a particular embodiment, the organic solvent is selected from chloroform, dichloromethane, dichloroethane, tetrachloroethane, cyclohexane, hexane(s), isooctane, dodecane, decane, methylbutyl ketone (MBK), methylcyclohexane, tetrahydrofuran, toluene, xylene, butyl acetate, mineral oil, *tert*-butylmethyl ether, heptanes(s), isobutyl acetate, isopropyl acetate, methyl acetate, methylethyl ketone, ethyl acetate, ethyl ether, pentane, and propyl acetate, or any suitably combination thereof.

In a particular embodiment, the organic solvent is selected from chloroform, dichloromethane, methylethylketone (MEK), methylbutylketone (MBK), and ethyl acetate.

The volume ratio of aqueous phase to oil phase in mixing step (iii) is suitably between 20:1 and 1:1, more suitably between 10:1 and 1:1, and most suitably between 6:1 and 2:1.

Mixing step (iii) suitably produces a substantially uniform oil-in-water emulsion. As previously indicated, mixing may be performed using methods well known in the art. Suitably, mixing step (iii) involves stirring, sonication, homogenisation, or a combination thereof. In a particular embodiment, mixing step (iii) involves sonication and/or homogenisation.

Removing the oil and water may be performed using methods well known in the art. Suitably removing the oil and water involves freeze-drying, spray-drying or spray-granulation.

Removing the oil and water may be performed using methods described in WO 2004/011537 A1 (COOPER et al)*.*

In a particular embodiment, removing the oil and water involves freeze drying the oil-in-water emulsion. Removing the oil and water may suitably comprise freezing the oil-in-water emulsion and then removing the solvents under vacuum.

Preferably, freezing of the oil-in-water emulsion may be performed by externally cooling the oil-in-water emulsion. For example, a vessel containing the oil-in-water emulsion may be externally cooled, by submerging the vessel in a cooling medium, such as liquid nitrogen. Alternative media for use for freezing purposes will be well known to those of skill in the art.

Alternatively the vessel containing the oil-in-water emulsion may be provided with an external "jacket" through which coolant is circulated to freeze the oil-in-water emulsion. Alternatively the vessel may comprise an internal element through which coolant is circulated in order to freeze the oil-in-water emulsion.

In a further alternative, the oil-in-water emulsion is frozen by being contacted directly with a cooling medium at a temperature effective for freezing the emulsion. The "temperature effective for freezing the emulsion" will be well understood by those of skill in the art based on the freezing temperature of the emulsion for freezing. It will be appreciated that the skilled person could readily determine what temperature would be so effective based on the freezing point of the emulsion and carrier materials thereof. In cases where the emulsion is frozen by contact with a cooling medium, the cooling medium (e.g. liquid nitrogen) may be added to the oil-in-water emulsion, or the oil-in-water emulsion may be added to the cooling medium.

In a particular embodiment, the oil-in-water emulsion is added to the fluid medium (e.g. liquid nitrogen), suitably in a dropwise manner, whereby frozen droplets of the oil-in-water emulsion may suitably form. This order of addition provides higher purities of final products. Frozen droplets may suitably be isolated (e.g. under vacuum to remove the fluid medium/liquid nitrogen). The solvent is then suitably removed from the frozen droplets under vacuum. The resulting solid composition is then isolated.

In an alternative aspect, the present invention provides a process for preparing a solid composition as defined herein, the process comprising:
- preparing a single phase solution comprising atovaquone, and one or more selected carrier materials, in one or more solvents; and
- removing the one or more solvents to form the solid composition.

In this aspect of the invention, the single phase solution comprising the atovaquone and one or more selected carrier materials are all dissolved in one solvent or two or more miscible solvents. Such processes are well described in WO2008/006712. WO2008/006712 also lists suitable solvents and combinations thereof for forming the single phase solution. In an embodiment, the single phase solution comprises two or more solvents (e.g. ethanol and water) which together solubilise atovaquone and the one or more selected carrier materials. In another embodiment, the single phase comprises a single solvent, for example ethanol or water.

Removing the one or more solvents may be performed using methods well known in the art. Suitably removing the one or more solvents involves freeze-drying, spray-drying or spray-granulation.

In a particular embodiment, removing the one or more solvents involves freeze drying the single phase solution. Removing the one or more solvents may suitably comprise freezing the single phase solution and then removing the solvents under vacuum.

The present invention also provides a solid composition obtainable by, obtained by, or directly obtained by any of the processes described herein.

### AQUEOUS OR OILY DISPERSION OF SOLID ATOVAQUONE NANOPARTICLES

The present invention provides an aqueous dispersion, comprising a plurality of nanoparticles of atovaquone dispersed in an aqueous medium, each nanoparticle of atovaquone being a core around at least some of which an outer layer composed of one or more carrier materials may be provided, wherein the atovaquone is present in a concentration of at least 10 mg/mL.

The present invention also provides an oily dispersion, comprising a plurality of nanoparticles of atovaquone and one or more carrier materials dispersed in an oily medium, wherein the atovaquone is present in a concentration of at least 10 mg/mL.

Preferably, or in one embodiment, the atovaquone is present in the dispersion in a concentration of at least 15 mg/mL. Further preferably, or an in alternative embodiment, the atovaquone is present in the dispersion in a concentration of at least 18 mg/mL. Yet further preferably, or in an alternative embodiment, the atovaquone is present in the dispersion in a concentration of at least 20 mg/mL or even at least 25 mg/mL.

The one or more solid carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

Accordingly, the one or more solid carrier material may be provided in any one or more of the following combinations:
- polyvinyl alcohol-polyethylene glycol graft copolymer AND polyoxyethylene (20) sorbitan monooleate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinyl alcohol AND sodium deoxycholate.

The one or more carrier materials are preferably water-soluble, such that they are dissolved and/or dispersed in an aqueous medium, and are able to form the outer layer around the individual cores of atovaquone, i.e. around each nanoparticle of atovaquone in the aqueous dispersions according to the invention. The aqueous medium may be, for example, sterile water.

The present invention also provides an aqueous dispersion, obtainable by, obtained by, or directly obtained by dispersing the solid composition as defined herein in an aqueous medium. Suitably, an aqueous dispersion is prepared immediately prior to use.

The present invention also provides an oily dispersion, obtainable by, obtained by, or directly obtained by dispersing the solid composition as defined herein in an oily medium. Suitably, an oily dispersion is prepared immediately prior to use.

When the solid composition is dispersed in the aqueous medium, the one or more carrier materials, e.g. providing the hydrophilic polymeric and surfactant activity, is/are dissolved within the aqueous medium to release the solid nanoparticles of atovaquone in a dispersed form. The nanoparticles of atovaquone, which were formerly dispersed within a solid mixture of the one or more selected carrier materials, then become dispersed within the aqueous medium in a form having an outer layer provided, whereby at least some of the cores of atovaquone are each individually "coated" with the one or more selected carrier materials. Such an outer layer is thought to impart stability to the nanoparticles, thereby preventing premature coagulation and aggregation.

When the solid composition is dispersed in the oily medium, the one or more carrier materials, e.g. providing the hydrophilic polymeric and surfactant activity, may be dissolved within the oily medium to release the solid nanoparticles of atovaquone in a dispersed form. The nanoparticles of atovaquone, which were formerly dispersed within a solid mixture of the one or more selected carrier materials, then become dispersed within the oily medium. Such a physical form may form one end of a continuum within which the physical form of the oily dispersion falls; the other end of the continuum being that the solid composition is comminuted, e.g. by grinding, to form smaller particles of the composition (of the one or more carrier materials having nanoparticles of atovaquone dispersed therein) which are dispersed in the oily medium such that the nanoparticles of atovaquone remain trapped in the matrix, i.e. the matrix per se does not dissolve.

Suitably the relative amounts (including ratios) of atovaquone, the carrier material(s) providing hydrophilic polymeric activity, and the carrier material(s) providing surfactant activity are the same as defined in relation to the solid composition. However, their respective wt% values in the dispersion as a whole must be adjusted to take account of the aqueous/oily medium. In a particular embodiment, the aqueous/oily medium comprises 20 to 99.5 wt% of the total dispersion. Preferably, or in a particular embodiment, the aqueous/oily medium may comprise 50 to 98 wt% of the total dispersion. Further preferably, or in a particular embodiment, the aqueous/oily medium may comprise 70 to 95 wt% of the total dispersion. Suitably, the remaining proportion of the dispersion consists essentially of atovaquone and the one or more selected carrier materials, whose proportions within the dispersion as a whole are accordingly calculated (and scaled) by reference to the proportions recited in relation to the solid composition.

In a particular embodiment, the aqueous medium is water, preferably sterile water. In an alternative embodiment, the aqueous medium comprises water and one or more additional pharmaceutically acceptable diluents or excipients.

In a particular embodiment, the oily medium is a non-aqueous medium, selected from the group including: squalene; natural oils, such as triglycerides; mineral oils; synthetic oils; vegetable oils, preferably avocado oil; rice bran oil; jojoba oil; Babassu oil; safflower seed oil; soybean oil; vitamin E; vitamin E acetate; non-vegetable oils such as silicone oils and paraffin oils; as well as waxes including carnauba wax, candelilla wax and lecithin. Mixtures of oils can be used. In an alternative embodiment, the oily medium is provided alongside one or more additional pharmaceutically acceptable diluents or excipients. In one particularly preferred embodiment, the oily medium is soybean oil.

Dispersions of the present invention are advantageously stable for prolonged periods, both in terms of chemical stability and the stability of the particles themselves (i.e. with respect to aggregation, coagulation, etc.).

Dispersions of the present invention may be considered as pharmaceutical compositions of the present invention. Thus, the dispersions of the present invention may be administered as is or further formulated with one or more additional excipients to provide a dispersion that is suitable for parenteral administration (for example, a sterile dispersion for intravenous, subcutaneous, intramuscular, or intraperitoneal dosing).

Dispersions of the present invention allow a measured aliquot to be taken therefrom for accurate dosing in a personalised medicine regime.

The particle size, polydispersity and zeta potential of the nanoparticles of atovaquone in the dispersions is as defined hereinbefore in relation to the solid composition. It will of course be appreciated that the particle size, polydispersity and zeta potential nanoparticles of atovaquone present in the solid composition are measured by dispersing the solid composition in an aqueous medium to thereby form an aqueous dispersion of the present invention.

### Process for Preparing an Aqueous Dispersion or an Oily Dispersion

The dispersion may be formed by methods well known in the art. For example, atovaquone may be milled in the presence of an aqueous/oily mixture of the one or more selected carrier materials.

In a particular aspect of the invention, however, there is provided a process for preparing an aqueous dispersion, comprising dispersing a solid atovaquone composition as defined herein in an aqueous medium.

In a particular embodiment, the aqueous medium is water. In an alternative embodiment, the aqueous medium comprises water and one or more additional excipients.

In a particular aspect of the invention, however, there is provided a process for preparing an oily dispersion, comprising dispersing a solid atovaquone composition as defined herein in an oily medium.

In a particular embodiment, the oily medium is soybean oil. In an alternative embodiment, the oily medium comprises soybean oil and one or more additional excipients.

Dispersing the solid composition in the aqueous/oily medium may comprise adding the solid composition to an aqueous/oily medium and suitably agitating the resulting mixture (e.g. by shaking, homogenisation, sonication, stirring, etc.).

### INJECTABLE FORMULATION OF ATOVAQUONE

The present invention also provides an intramuscularly-injectable formulation of nanoparticles of atovaquone.

The present invention also provides a subcutaneously-injectable formulation of nanoparticles of atovaquone.

Said formulations may be in solid form (or substantially solid form, e.g. a paste) or liquid form, in which the atovaquone is present in the form of nanoparticles. The nanoparticles of atovaquone may be dispersed within one or more carrier materials. When in liquid form, each nanoparticle of atovaquone may be provided as a core around which an outer layer composed of the one or more carrier materials is provided.

The one or more solid carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

Accordingly, the one or more solid carrier material may be provided in any one or more of the following combinations:
- polyvinyl alcohol-polyethylene glycol graft copolymer AND polyoxyethylene (20) sorbitan monooleate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinyl alcohol AND sodium deoxycholate.

The injectable formulations of nanoparticles atovaquone are advantageously designed for administration as a depot injection, so as to overcome the aforementioned problems associated with poor-adherence to prophylaxis and/or treatment, especially in respect of malaria, and the consequences that ensue. Furthermore, a depot injection is beneficial is that it may be easier to administer than conventional preparations and allows for simpler follow-up / on-going care.

Preferably, the injectable formulation of nanoparticles of atovaquone provides a controlled release bolus formulation of atovaquone, which, when administered to a patient (*via* intramuscular or subcutaneous injection), releases atovaquone into the bloodstream of the patient over a period of at least about two weeks from the date of administration. Further preferably the period of release is at least about three weeks, yet further preferably at least about one month, more preferably at least about three months, and most preferably at least about six months, from the date of administration of the injection.

### PHARMACEUTICAL OR VETERINARY COMPOSITION

The present invention provides a pharmaceutical or veterinary composition in injectable form comprising a solid composition as defined herein, and optionally one or more additional (pharmaceutically acceptable) excipients.

Preferably, said pharmaceutical or veterinary composition may be provided in intramuscularly-injectable or subcutaneously-injectable form.

Further preferably, said pharmaceutical or veterinary composition may be provided in depot form. The benefits of such a form are outlined earlier in this specification.

Preferably, the pharmaceutical or veterinary composition provides a controlled release bolus formulation of atovaquone, which, when administered to a patient in depot form releases atovaquone into the bloodstream of the patient over a period of at least about two weeks from the date of administration. Further preferably the period of release is at least about three weeks, yet further preferably at least about one month, more preferably at least about three months, and most preferably at least about six months, from the date of administration of the formulation.

In a particular embodiment, the pharmaceutical or veterinary composition is a dispersion as described herein. Such dispersed formulations can be used to accurately measure smaller dosages, such as those suitable for administration to children or small animals.

It will be appreciated that different pharmaceutical or veterinary compositions of the invention may be obtained by conventional procedures, using conventional (pharmaceutical) excipients, well known in the art.

The pharmaceutical or veterinary compositions of the invention contain a therapeutically effective amount of atovaquone. A person skilled in the art will know how to determine and select an appropriate therapeutically effective amount of atovaquone to include in the pharmaceutical or veterinary compositions of the invention.

### USES OF THE NANOPARTICLES FORMULATION AND PHARMACEUTICAL OR VETERINARY COMPOSITION

The present invention provides a solid composition, a dispersion, an injectable formulation, or a pharmaceutical or veterinary composition as defined herein, for use as a medicament. Said medicament may be used as a monotherapy or it may be combined with any one or more other drugs, such that a combination therapy of atovaquone with said one or more other drugs is provided. Such "other drugs" include, but are not limited to: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

In principle, the solid composition, dispersion, injectable formulation, or pharmaceutical or veterinary composition defined herein can be used for the treatment and/or propylaxis of a parasitic or fungal infection. Treatment and/or propylaxis of an infection such as infection by parasites of the genus *Plasmodium* is contemplated. Said infection, in particular, may be malaria. Treatment and/or prophylaxis of other infections, such as infections by parasites of the genus *Toxoplasma* (e.g. toxoplasmosis), by parasites of the genus *Babesiidae* (e.g. babesiosis), and infections by fungus of the genus *Pneumocystis* (e.g. *Pneumocystis* pneumonia) are also contemplated.

Said treatment and/or prophylaxis may be achieved using monotherapy or it may be that the atovaquone preparation is combined with any one or more other drugs, such that a combination therapy of atovaquone with said one or more other drugs is provided. Such "other drugs" include, but are not limited to: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

The present invention further provides a solid composition, dispersion, injectable formulation, or pharmaceutical or veterinary composition as defined herein for use in the treatment and/or prevention of malaria, toxoplasmosis, babesiosis and *Pneumocystis* pneumonia.

Said treatment and/or prevention may be achieved using monotherapy or it may be that the atovaquone preparation is combined with any one or more other drugs, such that a combination therapy of atovaquone with said one or more other drugs is provided. Such "other drugs" include, but are not limited to: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

The present invention further provides a use of a solid composition, dispersion, an injectable formulation, or pharmaceutical or veterinary composition as defined herein in the manufacture of a medicament for use in the treatment and/or prevention of parasitic and fungal infections (e.g. infection by parasites of the genus *Plasmodium,* particularly malaria, by parasites of the genus *Toxoplasma,* particularly toxoplasmosis, by parasites of the genus *Babesiidae,* particularly babesiosis, or infections by fungus of the genus *Pneumocystis,* particularly *Pneumocystis* pneumonia).

Said use may occur as a monotherapy or it may be that the atovaquone preparation is combined with any one or more other drugs, such that a combination therapy of atovaquone with said one or more other drugs is provided. Such "other drugs" include, but are not limited to: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

The present invention further provides a method of treating and/or preventing a parasitic or fungal infection (e.g. infection by parasites of the genus *Plasmodium,* particularly malaria, by parasites of the genus *Toxoplasma,* particularly toxoplasmosis, by parasites of the genus *Babesiidae,* particularly babesiosis, or infections by fungus of the genus *Pneumocystis,* particularly *Pneumocystis* pneumonia), the method comprising administering a therapeutically effective amount of a solid composition, a dispersion, an injectable formulation, or a pharmaceutical or veterinary composition as defined herein, to a patient suffering from or at risk of suffering from said infection (e.g. infection by parasites of the genus *Plasmodium,* particularly malaria, by parasites of the genus *Toxoplasma,* particularly toxoplasmosis, by parasites of the genus *Babesiidae,* particularly babesiosis, or infections by fungus of the genus *Pneumocystis,* particularly *Pneumocystis* pneumonia).

Said treatment and/or prophylaxis may be achieved using monotherapy or it may be that the atovaquone preparation is combined with any one or more other drugs, such that a combination therapy of atovaquone with said one or more other drugs is provided. Such "other drugs" include, but are not limited to: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

In particular, the present invention may be used to provide a method of preventing malaria by the administration of atovaquone by intramuscular injection at a dose of about 200 mg/kg. Preferably, such a method will provide a prophylactic effect for at least 28 days following administration. Alternatively the dose may be about 100 mg/kg and provide prophylaxis for at least 21 days. Alternatively the dose may be about 50 mg/kg and provide prophylaxis for at least 7 days. Further alternatively the dose may be about 35 mg/kg and provide prophylaxis for at least 7 days.

In any or all of the above-described uses, the administered form of nanoparticles of atovaquone preferably provides a controlled release bolus formulation of atovaquone, which, when administered to a patient, releases atovaquone into the bloodstream of the patient over a period of at least about two weeks from the date of administration. Further preferably the period of release is at least about three weeks, yet further preferably at least about one month, more preferably at least about three months, and most preferably at least about six months, from the date of administration of the injection.

### ROUTES OF ADMINISTRATION

The solid compositions, dispersions, and pharmaceutical or veterinary compositions of the invention, whether as a monotherapy or as a combination therapy (as hereinbefore discussed), may be administered to a patient by any convenient route of administration. More than one route of administration may be used in combination within a defined treatment and/or prophylactic regime, especially for a combination therapy, in which one component of the combination may be administered *via* one route, whilst another component of the combination may be administered *via* a different route. All such combinations are hereby contemplated.

Routes of administration include, but are not limited to, oral (e.g. by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, infraarterial, intracardiac, intrathecal, intraspinal, intracapsular, subcapsular, intraorbital, intraperitoneal, intratracheal, subcuticular, intraarticular, subarachnoid, and intrasternal; or by implant of a depot or reservoir, for example, subcutaneously or intramuscularly.

Most preferably, the route of administration is by parenteral implant of a depot or reservoir formulation.

Preferably, the injectable formulation of the present invention is a depot formulation administered so as to provide a controlled release formulation of atovaquone in the patient over at least a period of weeks, preferably months.

### FIGURES

The present invention will now be more particularly described, by way of non-limiting example only, with references to the accompanying figures, in which:
Figure 1 is a plot of the z-average particle sizes of nanoparticles of atovaquone achieved with the screen 'hits' described in Example 1;
Figure 2 is a plot of the z-average particle sizes of nanoparticles of atovaquone comparing a full-scale (standard) formulation with a half-scale formulation, as described in Example 1;
Figures 3A-3K are individual plots of the percentage of atovaquone released from various compositions according to the invention over a period of 6 hours, as compared to unformulated atovaquone, as described in Example 2;
Figure 4 is a plot of the percentage of atovaquone released by 24 hours for each of the compositions individually plotted in Figures 3A-3K, as compared to unformulated atovaquone, as described in Example 2;
Figures 5A-5C are individual plots of the percentage of atovaquone released from various compositions according to the invention over a period of 6 hours, as compared to unformulated atovaquone, as described in Example 4;
Figure 6 is a plot of the percentage of atovaquone released by 24 hours for each of the compositions individually plotted in Figures 5A-5C, as compared to unformulated atovaquone, as described in Example 4;
Figure 7 is a plot of the percentage atovaquone released from various oily dispersions according to the invention over a period of 6 hours, as compared to unformulated atovaquone, as described in Example 5;
Figures 8a and 8b are plots of the results of experiments to determine the efficacy of nanoformulations of atovaquone for providing anti-malarial prophylaxis in mice over a 42-day period for a variety of dosing regimens and dose-challenge intervals for ACS_ATQ_7 and ACS_ATQ_8 respectively, as described in Example 6;
Figures 9a and 9b are pharmacokinetic plots showing the variation in plasma concentration over time of ACS_ATQ_7 and ACS_ATQ_8 respectively following administration by intramuscular injection of 200 mg/kg of the nanoformulations to mice, as described in Example 7; and
Figure 10 is a plot showing the correlation between the plasma concentration of atovaquone and the efficacy of protection afforded.

### EXAMPLES

All materials were purchased and used without further purification from Sigma-Aldrich or Fisher Scientific unless specified otherwise.

### Example 1 - Screening for Nanoformulations of Atovaquone

Samples were prepared using an 80 mg/mL stock solution of atovaquone (A) in chloroform, a 22.5 mg/mL stock solution of polymer (P) and a 22.5 mg/mL stock solution of surfactant (S), both stock solutions being in water. Stock solutions were added in the following proportions: 100 µL (A); 63 µL (P) and 32 µL (S) (the solid mass ratio therefore being: 80 % (A); 13 % (P) and 7 % (S) in an 1:4 oil to water (O/W) mix). The mixtures were then emulsified using a Covaris S2x for 30 seconds with a duty cycle of 20, an intensity of 10 and 500 cycles/burst in frequency sweeping mode. Immediately after emulsification, the samples were cryogenically frozen.

A matrix of 42 samples was prepared. Once all 42 samples had been prepared, they were lyophilised (Virtis benchtop K) for 42 hours to leave a dry porous product; the samples were then sealed in individual vials until analysis.

The polymers and surfactants employed in this screen are detailed in Table 1A and Table 1B below:

**TABLE 1A - List of 7 hydrophilic polymers initially screened**

| **Polymer** | **MW** | **m/dm³ (22.5 mg/mL)** |
|---|---|---|
| **PEG 1K** | 1000 | 0.00225 |
| **Pluronic^{™} F68** | 8400 | 0.000267857 |
| **Pluronic^{™} F127** | 12600 | 0.000178571 |
| **Kollicoat^{™}** | 45000 | 0.00005 |
| **PVA** | 9500 | 0.000236842 |
| **PVP k30** | 30000 | 0.000075 |
| **HPMC** | 10000 | 0.000225 |

**TABLE 1B - List of 6 surfactants initially screened**

| **Surfactant** | **MW** | **m/dm³ (22.5 mg/mL)** |
|---|---|---|
| **NDC*** | 414.55 | 0.005427572 |
| **Vit E-peg-succinate**** | 1000 | 0.00225 |
| **AOT** | 444.56 | 0.005061184 |
| **Solutol^{™} HS 15***** | 344.53 | 0.006530636 |
| **Tween^{™} 20** | 1227 | 0.001833741 |
| **Tween^{™} 80** | 1300 | 0.001730769 |

| | | |
|---|---|---|
| ** NDC is sodium deoxycholate.* ** *Vit-E-PEG-succinate, also known as Vitamin E-TPGS is D-α-Tocopherol polyethylene glycol 1000 succinate.* *** *Solutol^{™} HS 15, also known as Kolliphor^{™} HS 15, is polyethylene glycol (15)-hydroxystearate.* | | |

All combinations of one polymer and one surfactant were tested (42 combinations total).

### Screen Analysis

Immediately prior to analysis, samples were dispersed by addition of 16 mL of water. The particle size of the active, organic nanoparticulate dispersion was then measured by dynamic light scattering (DLS) using a Malvern Zetasizer Nano ZS. Three measurements using automatic measurement optimisation, Malvern Zetasizer software version 7.11 was used for data analysis. The particles were considered hits if the below criteria were met.

### Nanodispersion Quality Assessment Criteria

A particle was determined a hit if it complied with the following criteria:
(i) complete dispersion of the sample with no large particles visible;
(ii) a particle Z-average <1000 nm;
(iii) a polydispersity index (PDI) <0.4;
(iv) a standard deviation between three scans <5% from average Z-average; and
(v) at least two of the three DLS scans pass the 'size quality report'.

The 'size quality report' incorporates twelve tests on the reliability of the data recorded and is automatically applied to each measurement by the Malvern Zetasizer software. These tests ensure that the sample is within a size range appropriate for DLS, has a PDI below 1, is within the correct concentration range and that the cumulant and distribution fit are good (i.e. the errors on the data are less than 0.005).

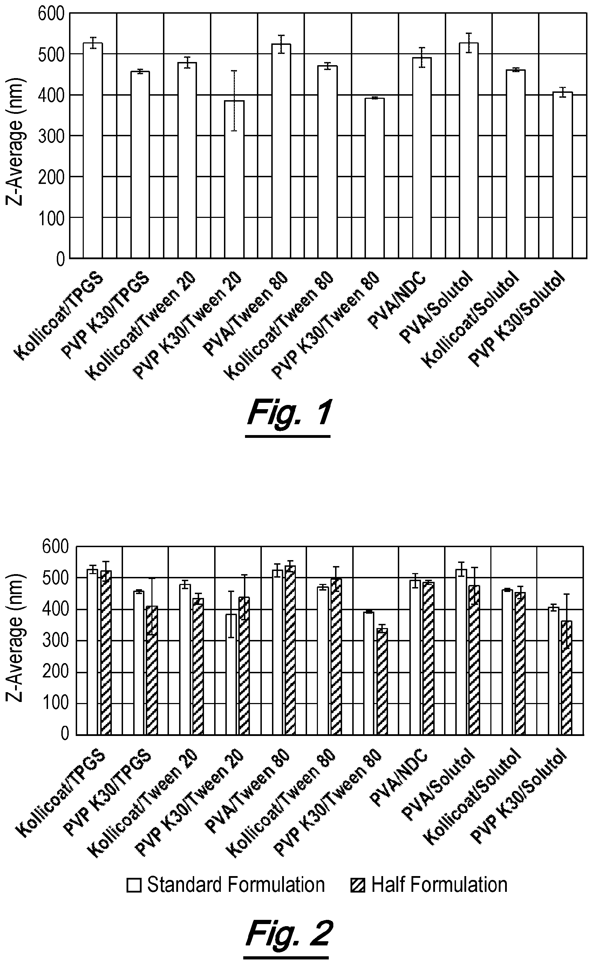

There were eleven hits that passed the selection criteria in the first screen which, upon repeating the screen, also passed the second screen. These eleven were selected for further study. The eleven hits were remade in replicate with samples prepared to be characterised by DLS and zeta potential analysis to show reproducibility (Figure 1; error bars are ±1 S.D.) while additional samples were prepared for biological assay to be carried out.

For pharmacological assessment the eleven formulation hits were to be prepared incorporating tritium-labelled atovaquone. However, in order to incorporate a suitable amount of radioactivity into the formulations, the formulations were to be prepared at half-scale. An initial comparison of non-labelled half-scale formulations with non-labelled full-scale formulations showed that the half-scale formulations produced similar size particles to the full-scale formulations, as shown in Figure 2 (error bars are ±1 S.D.).

Subsequently, radiolabelled particles were prepared following the same procedure for the half-scale preparations, but including ³H atovaquone in the atovaquone stock solution. The samples were prepared at 7.4 kBq/mg (0.2 µCi/mg) with respect to the drug. The prepared samples were characterised by DLS and zeta potentials were measured, shown in the Table 2 below.

**Table 2**

| **Sample** | **(A) (wt%)** | **(P)** | | **(S)** | | **Dz (nm)** | **Pdl** | **ζ-P (mV)** |
|---|---|---|---|---|---|---|---|---|
| | | **Name** | **(wt%)** | **Name** | **(wt%)** | | | |
| ACS_ATQ_1 | 80 | Kollicoat | 13 | TPGS | 7 | 477.0 | 0.281 | 8.16 |
| ACS_ATQ_2 | 80 | PVP k30 | 13 | TPGS | 7 | 364.4 | 0.261 | -19.5 |
| ACS_ATQ_3 | 80 | Kollicoat | 13 | Tween 20 | 7 | 440.0 | 0.322 | -16.3 |
| ACS_ATQ_4 | 80 | PVP k30 | 13 | Tween 20 | 7 | 388.4 | 0.284 | -16.4 |
| ACS_ATQ_5 | 80 | PVA | 13 | Tween 80 | 7 | 525.7 | 0.369 | -20.4 |
| ACS_ATQ_6 | 80 | Kollicoat | 13 | Tween 80 | 7 | 453.7 | 0.309 | -12.8 |
| ACS_ATQ_7 | 80 | PVP k30 | 13 | Tween 80 | 7 | 298.4 | 0.296 | -16.4 |
| ACS_ATQ_8 | 80 | PVA | 13 | NDC | 7 | 444.8 | 0.345 | -12.2 |
| ACS_ATQ_9 | 80 | PVA | 13 | Solutol | 7 | 517.3 | 0.352 | -15.5 |
| ACS_ATQ_10 | 80 | Kollicoat | 13 | Solutol | 7 | 439.6 | 0.332 | -17.0 |
| ACS_ATQ_11 | 80 | PVP k30 | 13 | Solutol | 7 | 383.9 | 0.337 | -17.2 |

| | | | | | | | | |
|---|---|---|---|---|---|---|---|---|
| Dz = Z-average diameter; Pdl = polydispersity index; ζ-P = zeta potential. | | | | | | | | |

### Example 2 - Assessment of AtovaquoneRelease from Solid Compositions

The release rate of atovaquone from solid compositions thereof in accordance with the invention, alongside an equivalent unformulated preparation of atovaquone, was assessed using Rapid Equilibrium Dialysis (RED) (ThermoFisher Scientific).

The eleven 'hit' formulations were dispersed in Simulated Interstitial Fluid (SIF) and subsequently diluted to 1 mg/mL of atovaquone. The SIF consisted of 35 mg/mL bovine serum albumin (BSA) and 2 mg/mL γ-Globulin derived from bovine blood and dissolved in distilled water. Unformulated atovaquone was initially dissolved in DMSO prior to dilution with SIF, such that the DMSO volume was <1% of the total volume.

Following preparation of the above suspensions, 0.5 mL of each was added to the donor compartment of the 8 kDa MWCO RED insert and 1 mL SIF was added to the acceptor compartment. The plates containing the RED inserts were subsequently incubated at 37°C, 100 rpm for 24 hours, prior to 0.5 mL of the acceptor contents being sampled and replaced with fresh pre-warmed (to 37°C) SIF at 0.5, 1, 2, 3, 4, 5, 6 and 24-hour time points.

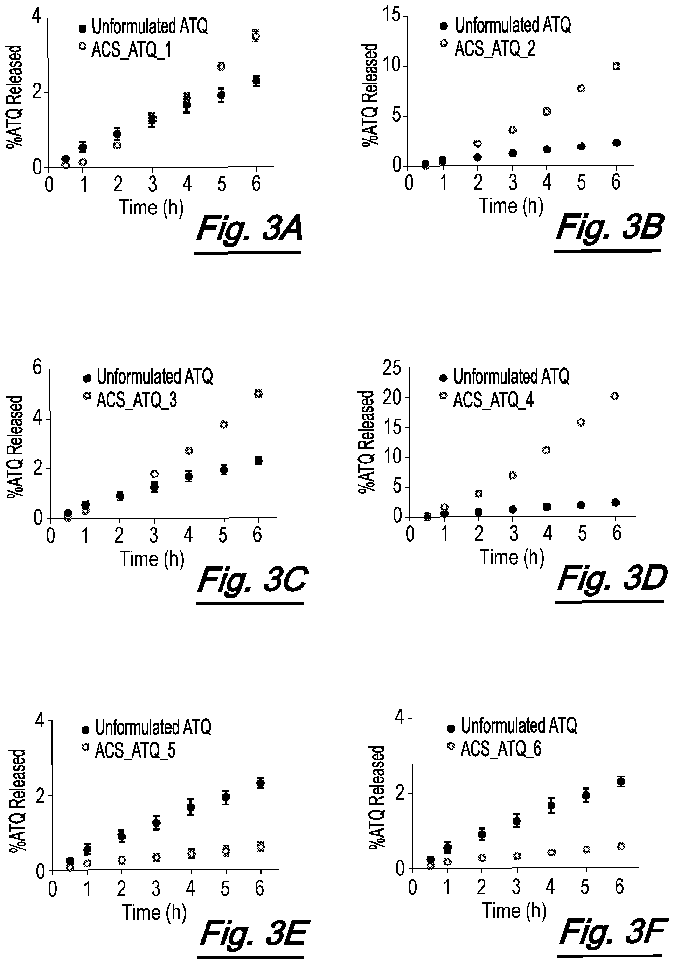

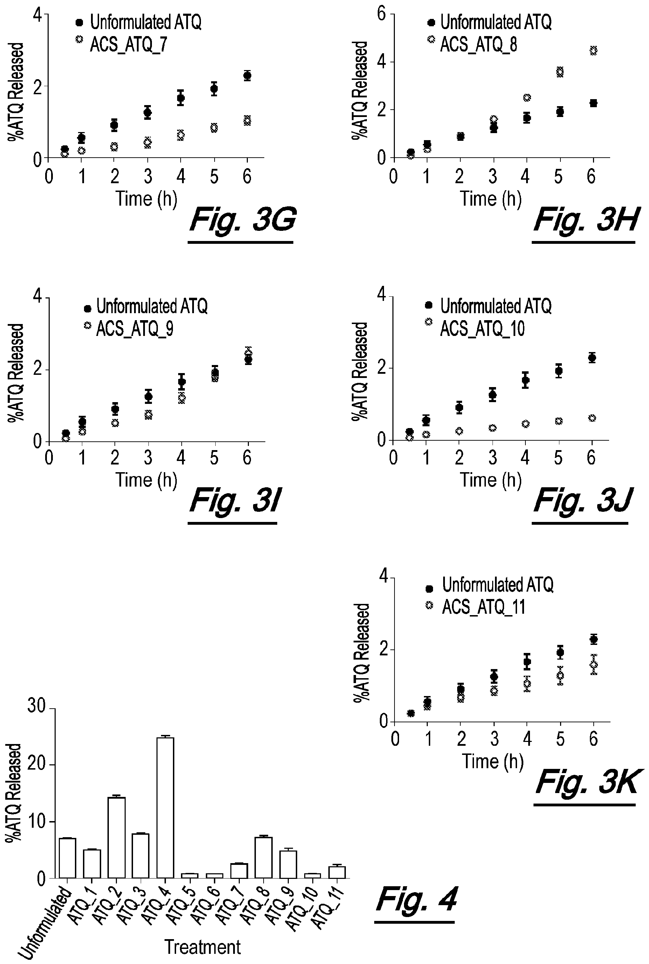

Following incubation, 4 mL ultima gold liquid scintillation cocktail fluid (Meridian Biotechnologies, UK) was added to each 0.1 mL sample and radioactivity was determined as disintegrations per minute (DPM) using a Perkin Elmer 3100TS scintillation counter. Data is expressed as the amount of atovaquone released and diffused across the membrane as a percentage of the starting donor amount, in triplicate, for each of the eleven hit formulations as compared to unformulated atovaquone, as shown in Figures 3A-3K (error bars are ±1 S.D.) and Figure 4 (release after 24 hours) (error bars are ±1 S.D.).

The release rate of atovaquone over the initial 6-hour period (shown in Figures 3A-3K) is provided in Table 3 below.

**Table 3**

| **Preparation** | **Release Rate (h⁻¹)** |
|---|---|
| Unformulated Atovaquone | 0.006 |
| ACS_ATQ_1 | 0.0112 |
| ACS_ATQ_2 | 0.0324 |
| ACS_ATQ_3 | 0.017 |
| ACS_ATQ_4 | 0.069 |
| ACS_ATQ_5 | 0.0014 |
| ACS_ATQ_6 | 0.0013 |
| ACS_ATQ_7 | 0.0031 |
| ACS_ATQ_8 | 0.0151 |
| ACS_ATQ_9 | 0.0095 |
| ACS_ATQ_10 | 0.0016 |
| ACS_ATQ_11 | 0.0038 |

### Example 3 - Testing of AtovaquoneNanoformulations in Murine Malaria Model

Various formulations of atovaquone identified in Example 2 were studied in a mouse model of *Plasmodium berghei.*

A murine malaria model consisting of C57BL6 male mice (6wks old, ~20g) infected with *Plasmodium berghei* (ANKA 2.34) strain was utilized. Sporozoite forms of the parasite were obtained from the salivary glands of infected *Anopheles stephensi* mosquitos.

The experimental strategy was to test atovaquone nanoformulations dosed intramuscularly at one day (Day -1 prophylaxis) or seven days (Day -7 prophylaxis) before an intravenous sporozoite challenge on Day 0, for their ability to protect mice. Parasites in tail snip blood samples constituted drug failure. Animals were monitored for up to 42 days after challenge.

All nanoformulations contained 80% by weight atovaquone. Excipients and first order release rate coefficients are listed in Table 4 below. Mepron^{™} (available from GSK and containing 150 mg/mL atovaquone) was dosed orally as an unformulated comparator and 10% Poloxamer 188 (Pluronic^{™} F-68) was used as an oral vehicle control.

**Table 4**

| **Sample** | **(P) (13wt%)** | **(S) (7 wt%)** | **1st Order Release Rate Coefficient (k, h⁻¹)** |
|---|---|---|---|
| ACS_ATQ_2 | PVP K30 | TPGS | 0.0324 |
| ACS_ATQ_4 | PVP K30 | Tween 20 | 0.069 |
| ACS_ATQ_6 | Kollicoat | Tween 80 | 0.0013 |
| ACS_ATQ_7 | PVP K30 | Tween 80 | 0.0031 |
| ACS_ATQ_8 | PVA | NDC | 0.0151 |

### Drug Dosing

Nanoformulations were dosed at 36 mg/kg in a total volume of 40 µL per 20 g animal. The dose was administered intramuscularly in two injections of 20 µL each. Doses were prepared by re-suspending lyophilized nanoformulations in purified sterile water to yield required concentration. The oral atovaquone control (Mepron^{™}) was diluted in 10% poloxamer 188 (Pluronic^{™} F-68) and 36 mg/kg was administered by gavage in a 200 µL volume. Food was withdrawn from all animals approximately 16 h before dosing and restored 4-6 h post dose.

### Sporozoite Challenge

*Anopheles stephensi* mosquitoes 4-6 day old that had been starved for 6 hr were fed on Swiss Webster mice infected with *Plasmodium berghei* (ANKA 2.34 strain). Fed mosquitoes were maintained at 19°C and 80% relative humidity. At 18 days post infection, mosquito salivary glands were dissected into RPMI medium and homogenized by passage several times through a 28 1/2 G needle, to release sporozoites. The sporozoites were counted by hemocytometer and light microscopy, and diluted to 25,000/mL RPMI. At challenge, 5,000 sporozoites in 200 µL RPMI were given intravenously. Mice were allocated in cohorts of three. This method consistently yielded detectable blood stage parasitemia by Day 3 post infection, and infected animals succumbed to malaria within 7 days post infection.

### Pharmacodynamic Endpoint

Infected animals were monitored for patent blood stage parasitemia *via* Giemsa-stained thin smears of tail snip blood samples examined by light microscopy. Sampling was initiated 72 h post-challenge and repeated every 48-72 h. Mice without detectable blood stage parasitemia at 42 days post-challenge were considered protected.

### Results

All three nanoformulations tested for Day -1 prophylaxis were protective, as was the oral atovaquone control. However, the oral atovaquone control was ineffective when dosed 7 days before challenge (day -7 prophylaxis). Samples ACS_ATQ_4 and ACS_ATQ_6 were also ineffective in this regimen. However, Sample ACS_ATQ_2 demonstrated partial efficacy, protecting 4 out of 6 animals and Samples ACS_ATQ_7 and ACS_ATQ_8 yielded complete and consistent protection with Day -7 prophylaxis. These results are provided in Table 5 below.

**Table 5**

| **Sample** | **Day -1 Prophylaxis** | **Day -7 Prophylaxis** |
|---|---|---|
| Mepron^{™} | **Protected** | Failed |
| ACS_ATQ_2 | **Protected** | **Limited Protection** (4/6) |
| ACS_ATQ_4 | Not Tested* | Failed |
| ACS_ATQ_6 | Not Tested* | Failed |
| ACS_ATQ_7 | **Protected** | **Protected** |
| ACS_ATQ_8 | **Protected** | **Protected** |

| | | |
|---|---|---|
| * *the data in Table 5 are for a dose of 36 mg*/*kg, however initial experiments with doses of 3 mg*/*kg showed that samples ACS_ATQ_2, ACS_ATQ_4 and ACS_ATQ_6 were all protective at day -1; given their efficacy on day -1, samples ACS_ATQ_4 and ACS_ATQ_6 were not retested at 36 mg*/*kg against challenge at day -1 to preserve test material.* | | |

### Example 4 - Assessment of Atovaquone Release from Solid Compositions with Different Loadings of Atovaquone

The release rate of atovaquone from solid compositions thereof in accordance with the invention having different loadings of atovaquone, alongside an equivalent unformulated preparation of atovaquone, was assessed using Rapid Equilibrium Dialysis (RED) (ThermoFisher Scientific).

Samples ACS_ATQ_4, ACS_ATQ_6 and ACS_ATQ_8 - already formulated with 80 wt% of atovaquone - were each made with 20 wt%, 40 wt% and 60 wt% loadings of atovaquone.

Samples were prepared using a 5 mg/mL stock solution of Atovaquone (A) in chloroform, a 22.5 mg/mL stock solution of polymer (P) and a 22.5 mg/mL stock solution of surfactant (S), both stock solutions being in water. Stock solutions were added in the following proportions: 100 µL (A); 63 µL (P) and 32 µL (S) (the solid mass ratio therefore being: 20% (A), 52% (P) and 28% (S) in an 1:4 oil to water (O/W) mix). The mixtures were the emulsified using a Covaris S2x for 30 seconds with a duty cycle of 20, an intensity of 10 and 500 cycles/burst in frequency sweeping mode. Immediately after emulsification, the samples were cryogenically frozen, and then lyophilised (Virtis benchtop K) for 42 hours to leave a dry porous product. The samples were then sealed in individual vials until analysis by DLS. Atovaquone loading (for the 40 wt% and 60 wt% samples) is varied by adjusting the concentration of the atovaquone stock solution, then following the same procedure given above.

The prepared samples were characterised by DLS, shown in the Table 6 below.

**Table 6**

| **Sample** | **(A) (wt%)** | **(P)** | | **(S)** | | **Dz (nm)** | **Pdl** |
|---|---|---|---|---|---|---|---|
| | | **Name** | **(wt%)** | **Name** | **(wt%)** | | |
| ATQ_4_20% | 20 | PVP k30 | 52 | Tween 20 | 28 | 505.4 | 0.151 |
| ATQ_4_40% | 40 | PVP k30 | 39 | Tween 20 | 21 | 406.5 | 0.222 |
| ATQ_4_60% | 60 | PVP k30 | 26 | Tween 20 | 14 | 439.2 | 0.322 |
| ATQ_6_20% | 20 | Kollicoat | 52 | Tween 80 | 28 | 668.5 | 0.226 |
| ATQ_6_40% | 40 | Kollicoat | 39 | Tween 80 | 21 | 467.9 | 0.236 |
| ATQ_6_60% | 60 | Kollicoat | 26 | Tween 80 | 14 | 510.4 | 0.302 |
| ATQ_8_20% | 20 | PVA | 52 | NDC | 28 | 890.8 | 0.199 |
| ATQ_8_40% | 40 | PVA | 39 | NDC | 21 | 523.6 | 0.322 |
| ATQ_8_60% | 60 | PVA | 26 | NDC | 14 | 437.7 | 0.256 |

| | | | | | | | |
|---|---|---|---|---|---|---|---|
| Dz = Z-average diameter; Pdl = polydispersity index. | | | | | | | |

Subsequently, radiolabelled particles were prepared following the same procedure as above, but including ³H atovaquone in the atovaquone stock solution. The samples were prepared at 7.4 kBq/mg (0.2 µCi/mg) with respect to the drug.

The nine (3 x 3) further radiolabelled formulations were dispersed in SIF and subsequently diluted to 1 mg/mL of atovaquone. The SIF consisted of 35 mg/mL BSA and 2 mg/mL γ-Globulin derived from bovine blood and dissolved in distilled water. Unformulated atovaquone was initially dissolved in DMSO prior to dilution with SIF, such that the DMSO volume was <1% of the total volume.

Following preparation of the above suspensions, 0.5 mL of each was added to the donor compartment of the 8 kDa MWCO RED insert and 1 mL SIF was added to the acceptor compartment. The plates containing the RED inserts were subsequently incubated at 37°C, 100 rpm for 24 hours, prior to 0.5 mL of the acceptor contents being sampled and replaced with fresh pre-warmed (to 37°C) SIF at 0.5, 1, 2, 3, 4, 5, 6 and 24-hour time points.

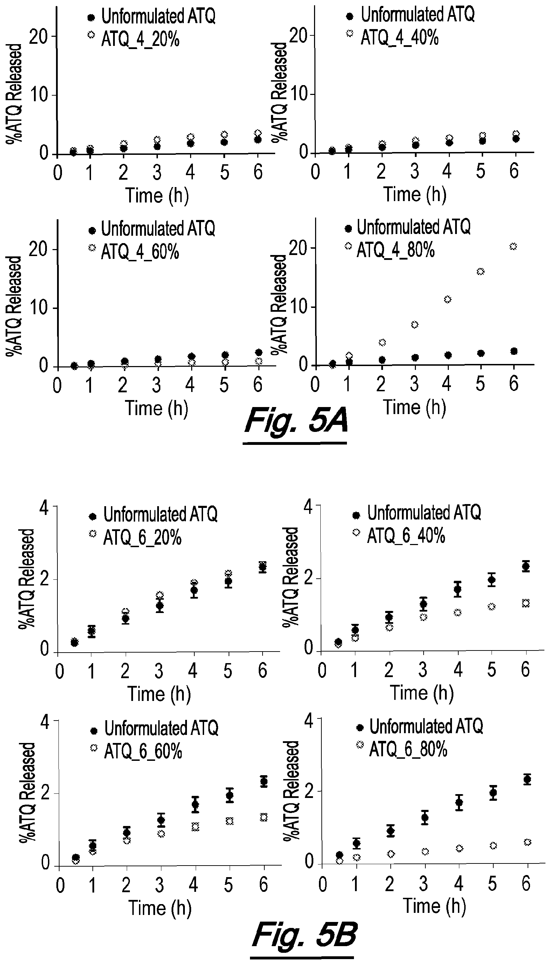

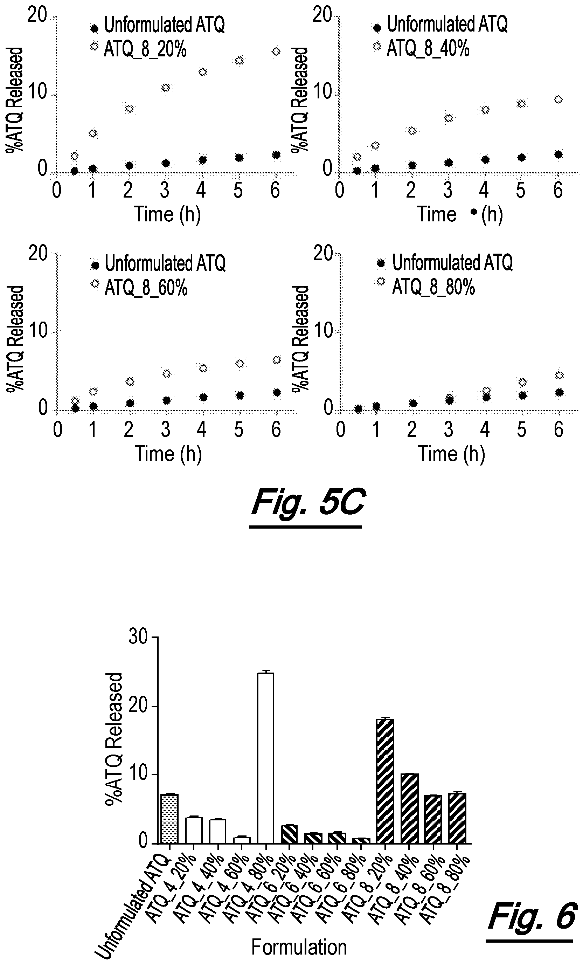

Following incubation, 4 mL ultima gold liquid scintillation cocktail fluid (Meridian Biotechnologies, UK) was added to each 0.1 mL sample and radioactivity was determined as disintegrations per minute (DPM) using a Perkin Elmer 3100TS scintillation counter. Data is expressed as the amount of atovaquone released and diffused across the membrane as a percentage of the starting donor amount, in triplicate, for each of the nine new formulations, along with the three existing 80 wt% formulations, as compared to unformulated atovaquone, as shown in Figures 5A-5C and Figure 6 (release after 24 hours) (error bars are ±1 S.D.).

The release rate of atovaquone over the initial 6-hour period (shown in Figures 5A-5C) is provided in Table 7 below.

**Table 7**

| **Preparation** | **Release Rate (h⁻¹)** |
|---|---|
| Unformulated Atovaquone | 0.0060 |
| ATQ_4_20% | 0.0088 |
| ATQ_4_40% | 0.0073 |
| ATQ_4_60% | 0.0020 |
| ATQ_4_80% | 0.0690 |
| ATQ_6_20% | 0.0063 |
| ATQ_6_40% | 0.0031 |
| ATQ_6_60% | 0.0030 |
| ATQ_6_80% | 0.0013 |
| ATQ_8_20% | 0.0348 |
| ATQ_8_40% | 0.0195 |
| ATQ_8_60% | 0.0153 |
| ATQ_8_80% | 0.0151 |

Clearly, there is variation in the percentage of atovaquone released in the 24-hour period studied (as shown in Figure 6) as well as variation in the release rate from each of the formulations tested (as shown in Table 7); with selection of appropriate atovaquone loading and combination of excipients, it is possible to design a release regimen according to a particular clinical need.

### Example 5 - Preparation of Paste Nanoformulations of Atovaquone(Oily Dispersions) for Release Studies

Solid compositions comprising nanoparticles of atovaquone were prepared following the same screening procedure as described in Example 1 above, except using 60 wt% atovaquone (A), 30 wt% of polymer (P) and 10 wt% of surfactant (S). Hits were assessed following the criteria listed in Example 1, and this screen identified a further eleven hits at this loading for further investigation. Three formulations (from the eleven) were chosen to study as a potential long-acting, controlled release depot injectable pastes.

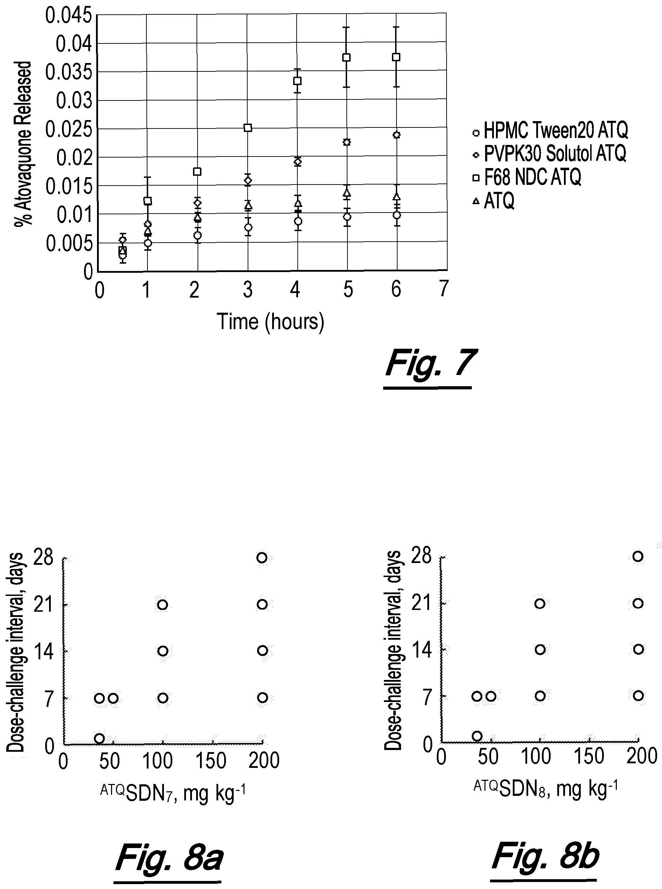

Pastes were prepared by blending the freeze-dried formulated monoliths with soybean oil. As noted earlier in this specification, the ratio of soybean oil to solid ingredients determines whether the oily dispersion is a liquid or, with a greater proportion of solid to oil, a paste. The release of atovaquone from these pastes into water was carried out using RED and UV-Vis spectroscopy. Unformulated atovaquone was also blended into a paste with soybean oil as a control. The release studies showed the presence of different polymers and surfactants within the formulations affects the release rate profile of the atovaquone into the water (as shown in Figure 7).

### Example 6 - Extended Testing of Atovaquone Nanoformulations in Murine Malaria Model

The test performed in Example 2 was extended in duration and scope for ACS_ATQ_7 and ACS_ATQ_8.

The experimental strategy was to test atovaquone nanoformulations dosed intramuscularly at one day (Day -1 prophylaxis), seven days (Day -7 prophylaxis), 14 days (Day -14 prophylaxis), 21 days (Day -21 prophylaxis) or 28 days (Day -28 prophylaxis) before an intravenous sporozoite challenge on Day 0, for their ability to protect mice. Parasites in tail snip blood samples constituted drug failure. Animals were monitored for up to 42 days after challenge.

All nanoformulations contained 80% by weight atovaquone. Excipients and first order release rate coefficients for ACS_ATQ_7 and ACS_ATQ_8 can be found in Table 4.

### Drug Dosing

The nanoformulations were dosed in the same manner as Example 3 but with concentrations of 50, 100 and 200 mg/kg in addition to the previously tested concentration of 36 mg/kg.

### Sporozoite Challenge

As for Example 3.

### Pharmacodynamic Endpoint

As for Example 3 the results are binary, if all mice remained parasite-free at 42 days after challenge then the mice are considered to be protected and the prophylaxis was successful. Conversely, prophylaxis was deemed to have failed if patent parasitemia was detected in any mouse in the dosing cohort.

### Results

Day -1 prophylaxis was again found to be successful for ACS_ATQ_7 and ACS_ATQ_8 at 36 mg/kg. The higher dosing regimens were not tested at this early time point as it was assumed that they would be effective. All dosing regimens tested for both ACS_ATQ_7 and ACS_ATQ_8 were found to provide effective Day -7 prophylaxis. Day -14 and Day -21 prophylaxis was only provided effectively by dosing regimens of 100 or 200 mg/kg for both ACS_ATQ_7 and ACS_ATQ_8. Only the highest doses of 200 mg/kg of ACS_ATQ_7 or ACS_ATQ_8 were found to provide effective Day -28 prophylaxis. These results are summarised in Table 8, as well as in Figures 8a and 8b for ACS_ATQ_7 and ACS_ATQ_8 respectively. Figures 8a and 8b plot successful dosing regimens and dose-challenge intervals (i.e. each circle displays a dosing regimen which provided successful prophylaxis at the corresponding dose-challenge interval).

**Table 8**

| Sample | Dose (mg/kg) | Day -1 prophylaxis | Day -7 prophylaxis | Day -14 prophylaxis | Day -21 prophylaxis | Day -28 prophylaxis |
|---|---|---|---|---|---|---|
| ACS_ATQ_7 | 36 | Protected | Protected | Failed | | |
| | 50 | | Protected | Failed | Failed | |
| | 100 | | Protected | Protected | Protected | Failed |
| | 200 | | Protected | Protected | Protected | Protected |
| ACS_ATQ_8 | 36 | Protected | Protected | Failed | | |
| | 50 | | Protected | Failed | Failed | |
| | 100 | | Protected | Protected | Protected | Failed |
| | 200 | | Protected | Protected | Protected | Protected |

The findings for ACS_ATQ_7 and ACS_ATQ_8 contrast with the results for oral atovaquone administered at 200 mg/kg and intramuscular injections of nanoformulation vehicles (i.e. polymer plus surfactant) which failed at Day -7 prophylaxis. The results also show that the duration of prophylaxis was dose dependent for both ACS_ATQ_7 and ACS_ATQ_8, with higher doses providing longer prophylaxis.

### Example 7 - Murine Pharmacokinetics of Intramuscular Injections of Atovaquone Nanoformulations

Mice were injected intramuscularly with 200 mg/kg of an atovaquone nanoformulation. At intervals blood was harvested (microtainer tubes, BD Biosciences), centrifuged (1300 x g, 10 min, 4 °C), and plasma was collected and stored at -80 °C until use. The nanoformulations tested were ACS_ATQ_7 and ACS_ATQ_8.

The concentration of atovaquone in the plasma was assayed by UPLC-MS/MS. Briefly, acetonitrile and deuterated atovaquone internal standard were added to 25 µL of thawed plasma, followed by filtration (Captiva filtration plate, Agilent Technologies). A 5 µL aliquot was separated on a reverse phase column and atovaquone was monitored by triple-quadrupole API4000 (SCIEX, Framingham, MA, USA) mass analyzer with electrospray ionization. The assay was validated to FDA guidelines for 250-50,000 ng/mL. Values between 250 and 77 ng/mL (the limit of detection) were interpreted conservatively.

### Results

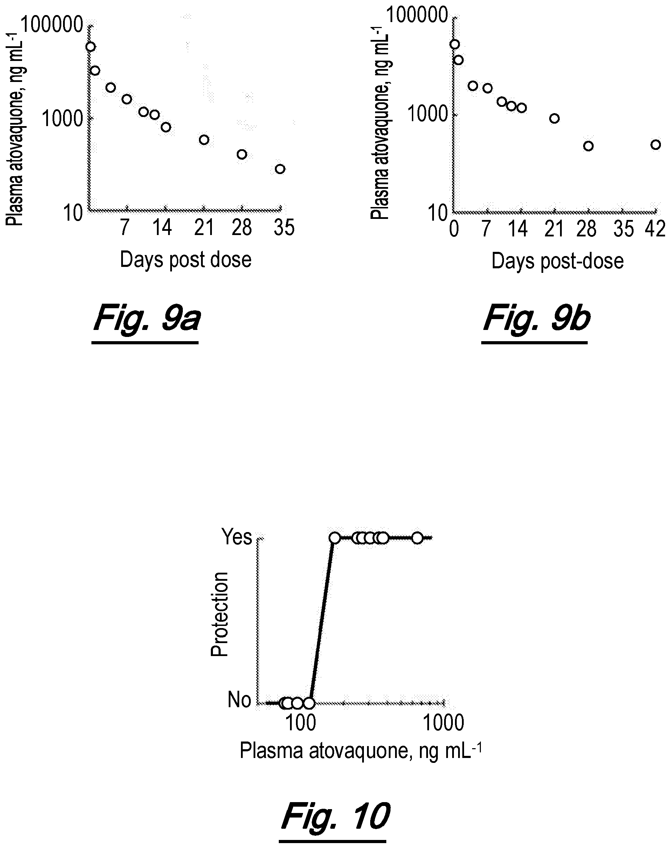

Atovaquone concentrations in mouse plasma were readily detected by 6h after intramuscular injection of ACS_ATQ_7 and the half-life was found to be 7 days, substantially longer than the 9 hour half-life known for orally dosed atovaquone in mice. The half-life for intramuscular injection of ACS_ATQ_8 was found to be 10.6 days. The results for these experiments for ACS_ATQ_7 and ACS_ATQ_8 are plotted in graphs in Figures 9a and 9b respectively.

From the findings of Examples 3, 6 and 7, and comparing plasma concentrations with efficacies that the Examples indicate, it can be seen that a plasma concentration of >200 ng/mL of atovaquone correlates with successful prophylaxis (Figure 10).

To conclude, murine malaria models demonstrated that nanoformulations of atovaquone provided effective malaria prophylaxis up to 28 days after intramuscular injection of the nanoformulation. Since the disposition of atovaquone in humans is approximately eight times slower than that in mice (plasma half-lives of 70 hours and 9 hours respectively), the duration of effective prophylaxis afforded by similarly administered nanoformulations of atovaquone in humans can be expected to extend beyond the 28 days observed in the murine model.

Atovaquone long-acting nanoformulations thus combine a safe, extensively studied, clinically used drug with excipients utilised in other FDA-approved medicines. The findings presented above suggest that a single intramuscular dose of nanoformulated atovaquone will provide causal prophylaxis against *P. falciparum* malaria for an extended period of time. This is an attractive option for non-immune people travelling to malarious areas, whose trips typically last 4 weeks or less, and if carefully deployed if may also provide a much-needed new intervention in malaria control efforts.

## Claims

1. A solid composition comprising nanoparticles of atovaquone dispersed within one or more carrier materials, wherein the atovaquone is present in an amount of at least 10 wt%, wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate; and
- polyvinyl alcohol AND polyethylene glycol (15)-hydroxystearate.

2. A solid composition as claimed in claim 1 wherein the one or more carrier materials provided in any one or more of the following combinations:
- polyvinyl alcohol-polyethylene glycol graft copolymer AND polyoxyethylene (20) sorbitan monooleate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinyl alcohol AND sodium deoxycholate.

3. A solid composition as claimed in claim 1 or claim 2, wherein:
(i) the nanoparticles of atovaquone have an average particle size between 100 and 800 nm as determined by dynamic light scattering; and/or
(ii) the polydispersity of the nanoparticles of atovaquone is less than or equal to 0.8 as determined by dynamic light scattering.

4. A process for preparing a solid composition according to any one of claims 1 to 3, the process comprising either (i) and (ii):
(i) preparing an oil-in-water emulsion comprising:
- an oil phase comprising atovaquone; and
- an aqueous phase comprising one or more selected carrier materials, the one or more selected carrier materials being defined in claim 1 or claim 2; and
(ii) removing the oil and water from the oil-in-water emulsion to form the solid composition; or
(iii) and (iv):
(iii) preparing a single phase solution comprising atovaquone and one or more selected carrier materials, the one or more selected carrier materials being defined in claim 1 or claim 2, in one or more solvents; and
(iv) removing the one or more solvents to form the solid composition, optionally wherein step (ii) or step (iv) comprises a freeze-drying step.

5. A pharmaceutical or veterinary composition in injectable form comprising a solid composition according to any one of claims 1 to 3, and optionally one or more additional (pharmaceutically acceptable) excipients, optionally wherein the pharmaceutical or veterinary composition is in intramuscularly-injectable and/or subcutaneously-injectable form.

6. An intramuscularly-injectable or subcutaneously-injectable formulation of nanoparticles of atovaquone, optionally wherein each nanoparticle of atovaquone is a core around which an outer layer composed of one or more carrier materials is provided,
wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

7. An intramuscularly-injectable and/or subcutaneously-injectable pharmaceutical or veterinary composition as claimed in claim 5, or an intramuscularly-injectable or subcutaneously-injectable formulation as claimed in claim 6, in depot form, optionally wherein, when administered to a patient releases atovaquone into the bloodstream of the patient over a period of at least about two weeks from the date of administration.

8. A dispersion comprising atovaquone in a concentration of at least of at least 10 mg/mL, which is either:
(i) an aqueous dispersion, comprising a plurality of nanoparticles of atovaquone dispersed in an aqueous medium, each nanoparticle of atovaquone being a core around at least some of which an outer layer composed of one or more carrier materials is provided; or
(ii) an oily dispersion, comprising a plurality of nanoparticles of atovaquone and one or more carrier materials dispersed in an oily medium,
wherein the one or more carrier materials are provided in any one or more of the following combinations:
- polyvinyl alcohol-polyethylene glycol graft copolymer AND D-α-tocopherol polyethylene glycol 1000 succinate;
- polyvinyl alcohol-polyethylene glycol graft copolymer AND polyoxyethylene (20) sorbitan monolaurate;
- polyvinyl alcohol-polyethylene glycol graft copolymer AND polyoxyethylene (20) sorbitan monooleate;
- polyvinyl alcohol-polyethylene glycol graft copolymer AND polyethylene glycol (15)-hydroxystearate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinylpyrrolidone k30 AND polyethylene glycol (15)-hydroxystearate;
- polyvinyl alcohol AND polyoxyethylene (20) sorbitan monooleate;
- polyvinyl alcohol AND sodium deoxycholate;
- polyvinyl alcohol AND polyethylene glycol (15)-hydroxystearate.

9. An intramuscularly-injectable formulation or a subcutaneously-injectable formulation as claimed in claim 6 or claim 7, or a dispersion as claimed in claim 8, wherein the one or more carrier materials are provided in any one or more of the following combinations:
- polyvinyl alcohol-polyethylene glycol graft copolymer AND polyoxyethylene (20) sorbitan monooleate;
- polyvinylpyrrolidone k30 AND D-α-tocopherol polyethylene glycol 1000 succinate;
- polyvinylpyrrolidone k30 AND polyoxyethylene (20) sorbitan monolaurate;
- polyvinylpyrrolidone k30 AND polyoxyethylene (20) sorbitan monooleate;
- polyvinyl alcohol AND sodium deoxycholate.

10. An intramuscularly-injectable formulation or a subcutaneously-injectable formulation as claimed in any one of claims 6 to 9, or a dispersion as claimed in claim 8 or claim 9:
(i) wherein each core consists essentially of atovaquone; and/or
(ii) wherein the nanoparticles of atovaquone have an average particle size between 100 and 800 nm as determined by dynamic light scattering; and/or
(iii) wherein the average zeta potential of the nanoparticles of atovaquone when dispersed in an aqueous medium is between -100 and +100 mV as determined by dynamic light scattering; and/or
(iv) comprising the atovaquone in a concentration of at least 10 mg/mL.

11. A process for preparing a dispersion according to any one of claims 8 to 10, comprising dispersing a solid composition according to any one of claims 1 to 3 in an aqueous medium or in an oily medium.

12. A solid composition according to any one of claims 1 to 3, a pharmaceutical or veterinary injectable composition according to any one of claims 5 or 7, an intramuscularly-injectable or subcutaneously-injectable formulation according to any one of claims 6, 7, 9 or 10, or a dispersion according to any one of claims 8 to 10, for use as a medicament, optionally wherein said use is as a monotherapy or, as a combination therapy by combination with any one or more of the following other drugs: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

13. A solid composition according to any one of claims 1 to 3, a pharmaceutical or veterinary injectable composition according to any one of claims 5 or 7, an intramuscularly-injectable or subcutaneously-injectable formulation according to any one of claims 6, 7, 9 or 10, or a dispersion according to any one of claims 8 to 10, for use in the treatment and/or prevention of parasitic and/or fungal infections, optionally wherein said use is as a monotherapy or, as a combination therapy by combination with any one or more of the following other drugs: proguanil, mefloquine, chloroquine, hydroxychloroquine, quinine, quinidine, artemether, lumefantrine, primaquine, doxycycline, tetracycline, clindamycin, dihydroartemisinin, piperaquine, and pyrimethamine with or without sulfadoxine.

14. A solid composition, a pharmaceutical or veterinary injectable composition, an intramuscularly-injectable or subcutaneously-injectable formulation, or a dispersion for the use of claim 13, wherein the parasitic infection is caused by parasites of the genus *Plasmodium*, or by parasites of the genus *Toxoplasma*, or by parasites of the genus *Babesiidae*, or wherein the fungal infection is caused by fungus of the genus *Pneumocystis.*

15. A solid composition, a pharmaceutical or veterinary injectable composition, an intramuscularly-injectable or subcutaneously-injectable formulation, or a dispersion for the use of claim 14, wherein the parasitic infection is malaria, toxoplasmosis, or babesiosis, or wherein the fungal infection is *Pneumocystis* pneumonia, optionally wherein the use is a method of preventing malaria, wherein:
(i) the atovaquone is administered by intramuscular injection at a dose of about 200 mg/kg; and/or
(ii) the prophylactic effect persists for at least 28 days after administration.

16. A kit for the preparation of a sterile liquid formulation of nanoparticles of atovaquone for injection, the kit comprising:
- a first container comprising a solid composition according to any one of claims 1 to 3, a pharmaceutical or veterinary injectable composition according to any one of claims 5 or 7, or an intramuscularly-injectable formulation or subcutaneously-injectable formulation according to any one of claims 6, 7, 9 or 10, and
- a second container comprising a sterile aqueous or oily diluent in an amount sufficient to dilute the atovaquone to a concentration of at least 10 mg/mL, optionally wherein:
(i) wherein the formulation is a depot formulation; and/or
(ii) the injection is an intramuscular injection or a subcutaneous injection.

## Patentansprüche

1. Feste Zusammensetzung umfassend Nanopartikel von Atovaquon, die innerhalb eines oder mehrerer Trägermaterialen dispergiert sind, wobei das Atovaquon in einer Menge von mindestens 10 Gew.-% vorliegt, wobei das eine oder die mehreren Trägermaterialien in einer oder mehreren der folgenden Kombinationen bereitgestellt sind:
- Polyvinylalkohol-Polyethylenglycol-Propfcopolymer UND D-α-Tocopherolpolyethylenglycol 1000-succinat;
- Polyvinylalkohol-Polyethylenglycol-Propfcopolymer UND Polyoxyethylen 20-Sorbitanmonolaurat;
- Polyvinylalkohol-Polyethylenglycol-Propfcopolymer UND Polyoxyethylen 20-Sorbitanmonooleat;
- Polyvinylalkohol-Polyethylenglycol-Propfcopolymer UND Polyethylenglycol 15-Hydroxystearat;
- Polyvinylpyrrolidon k30 UND D-α-Tocopherolpolyethylenglycol 1000-succinat;
- Polyvinylpyrrolidon k30 UND Polyoxyethylen 20-sorbitanmonolaurat;
- Polyvinylpyrrolidon k30 UND Polyoxyethylen 20-sorbitanmonooleat;
- Polyvinylpyrrolidon k30 UND Polyethylenglycol 15-hydroxystearat;
- Polyvinylalkohol UND Polyoxyethylen 20-sorbitanmonooleat;
- Polyvinylalkohol UND Natriumdesoxycholat; und
- Polyvinylalkohol UND Polyethylenglycol 15-hydroxystearat.

2. Feste Zusammensetzung nach Anspruch 1, wobei das eine oder die mehreren Trägermaterialien in einer oder mehreren der folgenden Kombinationen bereitgestellt sind:
- Polyvinylalkohol-Polyethylenglycol-Propfcopolymer UND Polyoxyethylen 20-sorbitanmonooleat;
- Polyvinylpyrrolidon k30 UND D-α-Tocopherolpolyethylenglycol 1000-succinat;
- Polyvinylpyrrolidon k30 UND Polyoxyethylen 20-sorbitanmonolaurat;
- Polyvinylpyrrolidon k30 UND Polyoxyethylen 20-sorbitanmonooleate;
- Polyvinylalkohol UND Natriumdesoxycholat.

3. Feste Zusammensetzung nach Anspruch 1 oder Anspruch 2, wobei:
(i) die Nanopartikel von Atovaquon eine durchschnittliche Partikelgröße zwischen 100 und 800 nm, wie durch dynamische Lichtstreuung bestimmt, aufweisen; und/oder
(ii) die Polydispersität der Nanopartikel von Atovaquon geringer als oder gleich 0,8, wie durch dynamische Lichtstreuung bestimmt, ist.

4. Verfahren für die Herstellung einer festen Zusammensetzung nach einem der Ansprüche 1 bis 3, wobei das Verfahren entweder (i) und (ii) umfasst:
(i) Herstellen einer Öl-in-Wasser-Emulsion, umfassend:
- eine Ölphase, die Atovaquon umfasst; und
- eine wässrige Phase, die ein oder mehrere ausgewählte Trägermaterialien umfasst, wobei das eine oder die mehreren ausgewählten Trägermaterialien in Anspruch 1 oder Anspruch 2 definiert sind; und
(ii) Entfernen des Öls und Wassers aus der Öl-in-Wasser-Emulsion, um die feste Zusammensetzung zu bilden; oder
(iii) und (iv):
(iii) Herstellen einer Einzelphaselösung, die Atovaquon und ein oder mehrere ausgewählte Trägermaterialien umfasst, wobei das eine oder die mehreren ausgewählten Trägermaterialien in Anspruch 1 oder Anspruch 2 definiert sind, in einem oder mehreren Lösungsmitteln; und
(iv) Entfernen des einen oder der mehreren Lösungsmittel, um die feste Zusammensetzung zu bilden, wobei wahlweise Schritt (ii) oder Schritt (iv) einen Gefriertrocknungsschritt umfasst.

5. Pharmazeutische oder veterinäre Zusammensetzung in injizierbarer Form, umfassend eine feste Zusammensetzung nach einem der Ansprüche 1 bis 3 und wahlweise einen oder mehrere zusätzliche (pharmazeutisch akzeptable) Trägerstoffe, wobei wahlweise die pharmazeutische oder veterinäre Zusammensetzung in intramuskulär injizierbarer und/oder subkutan injizierbarer Form vorliegt.

6. Intramuskulär injizierbare oder subkutan injizierbare Formulierung von Nanopartikeln von Atovaquon, wobei wahlweise jedes Nanopartikel von Atovaquon ein Kern ist, um den eine äußere Schicht, die aus einem oder mehreren Trägermaterialien besteht, bereitgestellt ist,
wobei das eine oder die mehreren Trägermaterialien in einer oder mehreren der folgenden Kombinationen bereitgestellt sind:
- Polyvinylalkohol-Polyethylenglycol-Propfcopolymer UND D-α-Tocopherolpolyethylenglycol 1000-succinat;
- Polyvinylalkohol-Polyethylenglycol-Propfcopolymer UND Polyoxyethylen 20-Sorbitanmonolaurat;
- Polyvinylalkohol-Polyethylenglycol-Propfcopolymer UND Polyoxyethylen 20-Sorbitanmonooleat;
- Polyvinylalkohol-Polyethylenglycol-Propfcopolymer UND Polyethylenglycol 15-Hydroxystearat;
- Polyvinylpyrrolidon k30 UND D-α-Tocopherolpolyethylenglycol 1000-succinat;
- Polyvinylpyrrolidon k30 UND Polyoxyethylen 20-sorbitanmonolaurat;
- Polyvinylpyrrolidon k30 UND Polyoxyethylen 20-sorbitanmonooleat;
- Polyvinylpyrrolidon k30 UND Polyethylenglycol 15-hydroxystearat;
- Polyvinylalkohol UND Polyoxyethylen 20-sorbitanmonooleat;
- Polyvinylalkohol UND Natriumdesoxycholat; und
- Polyvinylalkohol UND Polyethylenglycol 15-hydroxystearat.

7. Intramuskulär injizierbare und/oder subkutan injizierbare pharmazeutische oder veterinäre Zusammensetzung nach Anspruch 5 oder intramuskulär injizierbare oder subkutan injizierbare Formulierung nach Anspruch 6, in Depotform, wobei wahlweise, wenn einem Patienten verabreicht, sie Atovaquon in den Blutstrom des Patienten über eine Zeitspanne von mindestens zwei Wochen vom Verabreichungsdatum an, freigibt.

8. Dispersion umfassend Atovaquon in einer Konzentration von mindestens 10 mg/ml, die entweder:
(i) eine wässrige Dispersion ist, die eine Vielzahl von Nanopartikeln von Atovaquon umfasst, die in einem wässrigen Medium dispergiert sind, wobei jedes Nanopartikel von Atovaquon ein Kern ist, um mindestens einen Teil dessen eine äußere Schicht, die aus einem oder mehreren Trägermaterialien besteht, bereitgestellt ist; oder
(ii) eine ölige Dispersion, die eine Vielzahl von Nanopartikeln von Atovaquon und ein oder mehrere Trägermaterialien, die in einem öligen Medium dispergiert sind, umfasst,
wobei das eine oder die mehreren Trägermaterialien in einer oder mehreren der folgenden Kombinationen bereitgestellt sind:
- Polyvinylalkohol-Polyethylenglycol-Propfcopolymer UND D-α-Tocopherolpolyethylenglycol 1000-succinat;
- Polyvinylalkohol-Polyethylenglycol-Propfcopolymer UND Polyoxyethylen 20-Sorbitanmonolaurat;
- Polyvinylalkohol-Polyethylenglycol-Propfcopolymer UND Polyoxyethylen 20-Sorbitanmonooleat;
- Polyvinylalkohol-Polyethylenglycol-Propfcopolymer UND Polyethylenglycol 15-Hydroxystearat;
- Polyvinylpyrrolidon k30 UND D-α-Tocopherolpolyethylenglycol 1000-succinat;
- Polyvinylpyrrolidon k30 UND Polyoxyethylen 20-sorbitanmonolaurat;
- Polyvinylpyrrolidon k30 UND Polyoxyethylen 20-sorbitanmonooleat;
- Polyvinylpyrrolidon k30 UND Polyethylenglycol 15-hydroxystearat;
- Polyvinylalkohol UND Polyoxyethylen 20-sorbitanmonooleat;
- Polyvinylalkohol UND Natriumdesoxycholat;
- Polyvinylalkohol UND Polyethylenglycol 15-hydroxystearat.

9. Intramuskulär injizierbare Formulierung oder subkutan injizierbare Formulierung nach Anspruch 6 oder Anspruch 7 oder Dispersion nach Anspruch 8, wobei das eine oder die mehreren Trägermaterialien in einer oder mehreren der folgenden Kombinationen bereitgestellt sind:
- Polyvinylalkohol-Polyethylenglycol-Propfcopolymer UND Polyoxyethylen 20-sorbitanmonooleat;
- Polyvinylpyrrolidon k30 UND D-α-Tocopherolpolyethylenglycol 1000-succinat;
- Polyvinylpyrrolidon k30 UND Polyoxyethylen 20-sorbitanmonolaurat;
- Polyvinylpyrrolidon k30 UND Polyoxyethylen 20-sorbitanmonooleat;
- Polyvinylalkohol UND Natriumdesoxycholat.

10. Intramuskulär injizierbare Formulierung oder subkutan injizierbare Formulierung nach einem der Ansprüche 6 bis 9 oder Dispersion nach Anspruch 8 oder Anspruch 9:
(i) wobei jeder Kern im Wesentlichen aus Atovaquon besteht; und/oder
(ii) wobei die Nanopartikel von Atovaquon eine durchschnittliche Partikelgröße zwischen 100 und 800 nm, wie durch dynamische Lichtstreuung bestimmt, aufweisen; und/oder
(iii) wobei das durchschnittliche Zeta-Potential der Nanopartikel von Atovaquon, sind sie einem wässrigen Medium dispergiert, zwischen -100 und +100 mV, wie durch dynamische Lichtstreuung bestimmt, liegt; und/oder
(iv) umfassend das Atovaquon in einer Konzentration von mindestens 10 mg/ml.

11. Verfahren für die Herstellung einer Dispersion nach einem der Ansprüche 8 bis 10, das das Dispergieren einer feste Zusammensetzung nach einem der Ansprüche 1 bis 3 in einem wässrigen Medium oder in einem öligen Medium umfasst.

12. Feste Zusammensetzung nach einem der Ansprüche 1 bis 3, pharmazeutische oder veterinäre injizierbare Zusammensetzung nach einem der Ansprüche 5 oder 7, intramuskulär injizierbare oder subkutan injizierbare Formulierung nach einem der Ansprüche 6, 7, 9 oder 10 oder Dispersion nach einem der Ansprüche 8 bis 10 zur Verwendung als Medikament, wobei wahlweise die Verwendung eine Monotherapie ist, oder als Kombinationstherapie durch Kombination mit irgendeinem oder mehreren der folgenden anderen Arzneimittel erfolgt: Proguanil, Mefloquin, Chloroquin, Hydroxychloroquin, Chinin, Chinidin, Artemether, Lumefantrin, Primaquin, Doxycyclin, Tetracyclin, Clindamycin, Dihydroartemisinin, Piperaquin und Pyrimethamin mit oder ohne Sulfadoxin.

13. Feste Zusammensetzung nach einem der Ansprüche 1 bis 3, pharmazeutische oder veterinäre injizierbare Zusammensetzung nach einem der Ansprüche 5 oder 7, intramuskulär injizierbare oder subkutan injizierbare Formulierung nach einem der Ansprüche 6, 7, 9 oder 10 oder Dispersion nach einem der Ansprüche 8 bis 10 zur Verwendung bei der Behandlung und/oder Verhütung von parasitischen und/oder Pilzinfektionen, wobei wahlweise die Verwendung eine Monotherapie ist, oder als Kombinationstherapie durch Kombination mit irgendeinem oder mehreren der folgenden anderen Arzneimittel erfolgt: Proguanil, Mefloquin, Chloroquin, Hydroxychloroquin, Chinin, Chinidin, Artemether, Lumefantrin, Primaquin Doxycyclin, Tetracyclin, Clindamycin, Dihydroartemisinin, Piperaquin und Pyrimethamin mit oder ohne Sulfadoxin.

14. Feste Zusammensetzung, pharmazeutische oder veterinäre injizierbare Zusammensetzung, intramuskulär injizierbare oder subkutan injizierbare Formulierung oder Dispersion zur Verwendung nach Anspruch 13, wobei die parasitische Infektion durch Parasiten der Gattung *Plasmodium* oder Parasiten der Gattung *Toxoplasma* oder Parasiten der Gattung *Babesiidae* verursacht wird oder wobei die Pilzinfektionen durch Pilz der Gattung *Pneumocystis* verursacht wird.

15. Feste Zusammensetzung, pharmazeutische oder veterinäre injizierbare Zusammensetzung, intramuskulär injizierbare oder subkutan injizierbare Formulierung oder Dispersion zur Verwendung nach Anspruch 14, wobei die parasitische Infektion Malaria, Toxoplasmose oder Babesiose ist oder wobei die Pilzinfektion *Pneumocystis pneumonia* ist, wobei wahlweise die Verwendung ein Verfahren zur Prävention von Malaria ist, wobei:
(i) das Atovaquon durch intramuskuläre Injektion in einer Dosis von etwa 200 mg/kg verabreicht wird; und/oder
(ii) die prophylaktische Wirkung mindestens 28 Tage lang nach der Verabreichung andauert.

16. Kit für die Herstellung einer sterilen flüssigen Formulierung von Nanopartikeln von Atovaquon zur Injektion, wobei das Kit Folgendes umfasst:
- einen ersten Behälter, der eine feste Zusammensetzung nach einem der Ansprüche 1 bis 3, eine pharmazeutische oder veterinäre injizierbare Zusammensetzung nach einem der Ansprüche 5 oder 7 oder eine intramuskulär injizierbare Formulierung oder subkutan injizierbare Formulierung nach einem der Ansprüche 6, 7, 9 oder 10 umfasst und
- einen zweiten Behälter, der ein steriles wässriges oder öliges Verdünnungsmittel in einer Menge umfasst, die zum Verdünnen des Atovaquons auf eine Konzentration von mindestens 10 mg/ml ausreicht, wobei wahlweise:
(i) die Formulierung eine Depotformulierung ist; und/oder
(ii) die Injektion eine intramuskuläre Injektion oder eine subkutane Injektion ist.

## Revendications

1. Composition solide comprenant des nanoparticules d'atovaquone dispersées à l'intérieur d'un ou plusieurs matériaux supports, dans laquelle l'atovaquone est présent en une quantité d'au moins 10 % en pds, dans laquelle les un ou plusieurs matériaux supports sont fournis selon n'importe laquelle des une ou plusieurs des combinaisons suivantes :
- copolymère greffé de poly(alcool de vinyle-polyéthylène glycol) ET de succinate de D-α-tocophérol polyéthylène glycol 1000 ;
- copolymère greffé de poly(alcool de vinyle-polyéthylène glycol) ET de monolaurate de polyoxyéthylène (20) sorbitan ;
- copolymère greffé de poly(alcool de vinyle-polyéthylène glycol) ET de monooléate de polyoxyéthylène (20) sorbitan ;
- copolymère greffé de poly(alcool de vinyle-polyéthylène glycol) ET d'hydroxystéarate de polyéthylène glycol (15) ;
- polyvinylpyrrolidone k30 ET succinate de D-α-tocophérol polyéthylène glycol 1000 ;
- polyvinylpyrrolidone k30 ET monolaurate de polyoxyéthylène (20) sorbitan ;
- polyvinylpyrrolidone k30 ET monooléate de polyoxyéthylène (20) sorbitan ;
- polyvinylpyrrolidone k30 ET hydroxystéarate de polyéthylène glycol (15) ;
- poly(alcool de vinyle) ET monooléate de polyoxyéthylène (20) sorbitan ;
- poly(alcool de vinyle) ET désoxycholate de sodium ; et
- poly(alcool de vinyle) ET hydroxystéarate de polyéthylène glycol (15).

2. Composition solide selon la revendication 1, dans laquelle les un ou plusieurs matériaux supports est/sont fournis selon n'importe laquelle des une ou plusieurs des combinaisons suivantes :
- copolymère greffé de poly(alcool de vinyle-polyéthylène glycol) ET de monooléate de polyoxyéthylène (20) sorbitan ;
- polyvinylpyrrolidone k30 ET succinate de D-α-tocophérol polyéthylène glycol 1000 ;
- polyvinylpyrrolidone k30 ET monolaurate de polyoxyéthylène (20) sorbitan ;
- polyvinylpyrrolidone k30 ET monooléate de polyoxyéthylène (20) sorbitan ;
- poly(alcool de vinyle) ET désoxycholate de sodium.

3. Composition solide selon la revendication 1 ou la revendication 2, dans laquelle :
(i) les nanoparticules d'atovaquone présentent une taille moyenne de particule comprise entre 100 et 800 nm telle que déterminée par diffusion dynamique de la lumière ; et/ou
(ii) la polydispersité des nanoparticules d'atovaquone est inférieure ou égale à 0,8 telle que déterminée par diffusion dynamique de la lumière.

4. Procédé de préparation d'une composition solide selon l'une quelconque des revendications 1 à 3, le procédé comprenant soit (i) et (ii) :
(i) préparation d'une émulsion huile-dans-eau comprenant :
- une phase huileuse comprenant de l'atovaquone ; et
- une phase aqueuse comprenant un ou plusieurs matériaux supports sélectionnés, les un ou plusieurs matériaux supports sélectionnés étant définis selon la revendication 1 ou la revendication 2 ; et
(ii) retrait de l'huile et de l'eau de l'émulsion huile-dans-eau pour former la composition solide ; soit
(iii) et (iv) :
(iii) préparation d'une solution à phase unique comprenant de l'atovaquone et un ou plusieurs matériaux supports sélectionnés, les un ou plusieurs matériaux supports sélectionnés étant définis selon la revendication 1 ou la revendication 2, dans un ou plusieurs solvants ; et
(iv) retrait des un ou plusieurs solvants pour former la composition solide, facultativement dans lequel l'étape (ii) ou l'étape (iv) comprend une étape de cryodessiccation.

5. Composition pharmaceutique ou vétérinaire sous une forme injectable comprenant une composition solide selon l'une quelconque des revendications 1 à 3, et facultativement un ou plusieurs excipients additionnels (pharmaceutiquement acceptables), facultativement où la composition pharmaceutique ou vétérinaire se présente sous une forme injectable par voie intramusculaire et/ou injectable par voie sous-cutanée.

6. Formulation injectable par voie intramusculaire ou injectable par voie sous-cutanée de nanoparticules d'atovaquone, facultativement dans laquelle chaque nanoparticule d'atovaquone est un coeur autour duquel est fournie une couche externe composée d'un ou plusieurs matériaux supports,
dans laquelle les un ou plusieurs matériaux supports sont fournis selon n'importe laquelle ou lesquelles des combinaisons suivantes :
- copolymère greffé de poly(alcool de vinyle-polyéthylène glycol) ET de succinate de D-α-tocophérol polyéthylène glycol 1000 ;
- copolymère greffé de poly(alcool de vinyle-polyéthylène glycol) ET de monolaurate de polyoxyéthylène (20) sorbitan ;
- copolymère greffé de poly(alcool de vinyle-polyéthylène glycol) ET de monooléate de polyoxyéthylène (20) sorbitan ;
- copolymère greffé de poly(alcool de vinyle-polyéthylène glycol) ET d'hydroxystéarate de polyéthylène glycol (15) ;
- polyvinylpyrrolidone k30 ET succinate de D-α-tocophérol polyéthylène glycol 1000 ;
- polyvinylpyrrolidone k30 ET monolaurate de polyoxyéthylène (20) sorbitan ;
- polyvinylpyrrolidone k30 ET monooléate de polyoxyéthylène (20) sorbitan ;
- polyvinylpyrrolidone k30 ET hydroxystéarate de polyéthylène glycol (15) ;
- poly(alcool de vinyle) ET monooléate de polyoxyéthylène (20) sorbitan ;
- poly(alcool de vinyle) ET désoxycholate de sodium ;
- poly(alcool de vinyle) ET hydroxystéarate de polyéthylène glycol (15).

7. Composition pharmaceutique ou vétérinaire injectable par voie intramusculaire et/ou injectable par voie sous-cutanée selon la revendication 5, ou formulation injectable par voie intramusculaire ou injectable par voie sous-cutanée selon la revendication 6, sous une forme de dépôt, facultativement , lorsqu'administrée à un patient, libérant de l'atovaquone dans le flux sanguin du patient sur une période d'au moins environ deux semaines depuis la date d'administration.

8. Dispersion comprenant de l'atovaquone sous une concentration d'au moins d'au moins 10 mg/ml, qui est soit :
(i) une dispersion aqueuse, comprenant une pluralité de nanoparticules d'atovaquone dispersées dans un milieu aqueux, chaque nanoparticule d'atovaquone étant un coeur autour d'au moins une partie duquel une couche externe composée d'un ou plusieurs matériaux supports est fournie ; ou
(ii) une dispersion huileuse, comprenant une pluralité de nanoparticules d'atovaquone et un ou plusieurs matériaux supports dispersés dans un milieu huileux,
dans laquelle les un ou plusieurs matériaux supports sont fournis selon n'importe laquelle ou lesquelles des combinaisons suivantes :
- copolymère greffé de poly(alcool de vinyle-polyéthylène glycol) ET de succinate de D-α-tocophérol polyéthylène glycol 1000 ;
- copolymère greffé de poly(alcool de vinyle-polyéthylène glycol) ET de monolaurate de polyoxyéthylène (20) sorbitan ;
- copolymère greffé de poly(alcool de vinyle-polyéthylène glycol) ET de monooléate de polyoxyéthylène (20) sorbitan ;
- copolymère greffé de poly(alcool de vinyle-polyéthylène glycol) ET d'hydroxystéarate de polyéthylène glycol (15) ;
- polyvinylpyrrolidone k30 ET succinate de D-α-tocophérol polyéthylène glycol 1000 ;
- polyvinylpyrrolidone k30 ET monolaurate de polyoxyéthylène (20) sorbitan ;
- polyvinylpyrrolidone k30 ET monooléate de polyoxyéthylène (20) sorbitan ;
- polyvinylpyrrolidone k30 ET hydroxystéarate de polyéthylène glycol (15) ;
- poly(alcool de vinyle) ET monooléate de polyoxyéthylène (20) sorbitan ;
- poly(alcool de vinyle) ET désoxycholate de sodium ;
- poly(alcool de vinyle) ET hydroxystéarate de polyéthylène glycol (15).

9. Formulation injectable par voie intramusculaire ou formulation injectable par voie sous-cutanée selon la revendication 6 ou la revendication 7, ou dispersion selon la revendication 8, dans laquelle les un ou plusieurs matériaux supports sont fournis selon n'importe laquelle ou lesquelles des combinaisons suivantes :
- copolymère greffé de poly(alcool de vinyle-polyéthylène glycol) ET de monooléate de polyoxyéthylène (20) sorbitan ;
- polyvinylpyrrolidone k30 ET succinate de D-α-tocophérol polyéthylène glycol 1000 ;
- polyvinylpyrrolidone k30 ET monolaurate de polyoxyéthylène (20) sorbitan ;
- polyvinylpyrrolidone k30 ET monooléate de polyoxyéthylène (20) sorbitan ;
- poly(alcool de vinyle) ET désoxycholate de sodium.

10. Formulation injectable par voie intramusculaire ou formulation injectable par voie sous-cutanée selon l'une quelconque des revendications 6 à 9, ou dispersion selon la revendication 8 ou la revendication 9 :
(i) dans lesquelles chaque coeur est essentiellement constitué d'atovaquone ; et/ou
(ii) dans lesquelles les nanoparticules d'atovaquone présentent une taille moyenne de particule comprise entre 100 et 800 nm telle que déterminée par diffusion dynamique de la lumière ; et/ou
(iii) dans lesquelles le potentiel zêta moyen des nanoparticules d'atovaquone, lorsque dispersées dans un milieu aqueux, est compris entre -100 et +100 mV, tel que déterminé par diffusion dynamique de la lumière ; et/ou
(iv) comprenant l'atovaquone en une concentration d'au moins 10 mg/ml.

11. Procédé de préparation d'une dispersion selon l'une quelconque des revendications 8 à 10, comprenant la dispersion d'une composition solide selon l'une quelconque des revendications 1 à 3 dans un milieu aqueux ou dans un milieu huileux.

12. Composition solide selon l'une quelconque des revendications 1 à 3, composition pharmaceutique ou vétérinaire injectable selon l'une quelconque des revendications 5 ou 7, formulation injectable par voie intramusculaire ou injectable par voie sous-cutanée selon l'une quelconque des revendications 6, 7, 9 ou 10, ou dispersion selon l'une quelconque des revendications 8 à 10, pour l'utilisation comme médicament, facultativement où ladite utilisation est une monothérapie, ou comme polythérapie par combinaison de n'importe lequel ou de plusieurs des autres médicaments suivants : proguanil, méfloquine, chloroquine, hydroxychloroquine, quinine, quinidine, artéméther, luméfantrine, primaquine, doxycycline, tétracycline, clindamycine, dihydroartémisinine, pipéraquine, et pyriméthamine avec ou sans sulfadoxine.

13. Composition solide selon l'une quelconque des revendications 1 à 3, composition pharmaceutique ou vétérinaire injectable selon l'une quelconque des revendications 5 ou 7, formulation injectable par voie intramusculaire ou injectable par voie sous-cutanée selon l'une quelconque des revendications 6, 7, 9 ou 10, ou dispersion selon l'une quelconque des revendications 8 à 10, pour utilisation dans le traitement et/ou la prévention d'infections parasitaires et/ou fongiques, facultativement où ladite utilisation est une monothérapie, ou comme polythérapie par combinaison de n'importe lequel ou de plusieurs des autres médicaments suivants : proguanil, méfloquine, chloroquine, hydroxychloroquine, quinine, quinidine, artéméther, luméfantrine, primaquine, doxycycline, tétracycline, clindamycine, dihydroartémisinine, pipéraquine, et pyriméthamine avec ou sans sulfadoxine.

14. Composition solide, composition pharmaceutique ou vétérinaire injectable, formulation injectable par voie intramusculaire ou injectable par voie sous-cutanée, ou dispersion pour utilisation selon la revendication 13, où l'infection parasitaire est causée par des parasites du genre *Plasmodium*, ou par des parasites du genre *Toxoplasma*, ou par des parasites du genre *Babesiidae*, ou où l'infection fongique est causée par un champignon du genre *Pneumocystis.*

15. Composition solide, composition pharmaceutique ou vétérinaire injectable, formulation injectable par voie intramusculaire ou injectable par voie sous-cutanée, ou dispersion pour utilisation selon la revendication 14, où l'infection parasitaire est la malaria, la toxoplasmose, ou la babésiose, ou où l'infection fongique est la pneumonie à *Pneumocystis,* facultativement où l'utilisation est un procédé de prévention de la malaria, dans lequel :
(i) l'atovaquone est administré par injection intramusculaire à une dose d'environ 200 mg/kg ; et/ou
(ii) l'effet prophylactique persiste durant au moins 28 jours après l'administration.

16. Kit de préparation d'une formulation liquide stérile de nanoparticules d'atovaquone pour injection, le kit comprenant :
- un premier récipient comprenant une composition solide selon l'une quelconque des revendications 1 à 3, une composition pharmaceutique ou vétérinaire injectable selon l'une quelconque des revendications 5 ou 7, ou une formulation injectable par voie intramusculaire ou une formulation injectable par voie sous-cutanée selon l'une quelconque des revendications 6, 7, 9 ou 10, et
- un deuxième récipient comprenant un diluant aqueux ou huileux stérile en une quantité suffisante pour diluer l'atovaquone jusqu'à une concentration d'au moins 10 mg/ml, facultativement dans lequel :
(i) la formulation est une formulation en dépôt ; et/ou
(ii) l'injection est une injection intramusculaire ou une injection sous-cutanée.
